# DEMANDE DE BREVET EUROPEEN

(11) **EP 4 571 755 A1**
(43) Date de publication de la demande: **18.06.2025**
(21) Numéro de dépôt: 23307186.9
(22) Date de dépôt: 12.12.2023
(51) Int. Cl.: G16B 40/20

(54) **CLASSIFICATION DES HYPERCHOLESTEROLEMIES SEVERES**

(71) Demandeur: Assistance Publique - Hôpitaux de Paris, 75012 Paris (FR); Sorbonne Université, 75006 Paris (FR); Institut National de la Santé et de la Recherche Médicale, 75013 Paris (FR)
(72) Inventeur: BLUTEAU, Olivier, 75012 Paris (FR); CARRIE, Alain, 94220 Charenton-le-Pont (FR); GIRAL, Philippe, 75012 Paris (FR); LAPIDUS, Nathanael, 75012 Paris (FR)
(74) Mandataire: Novagraaf Technologies

(57) **Abrégé**

Un profil hypercholestérolémique d'un patient est déterminé sur la base (i) d'une première distribution statistique en fonction d'une échelle de niveaux de score de risque polygénique et d'une échelle de niveaux de concentration de cholestérol dans des lipoprotéines de basse densité, la première distribution statistique donnant en fonction de ces échelles une répartition des individus d'un premier sous-échantillon d'individus hypercholestérolémiques sévères présentant au moins une mutation génétique à l'origine d'une hypercholestérolémie pour au moins un gène parmi un ensemble de gènes et (ii) d'une deuxième distribution statistique en fonction de la même échelle de niveaux du score de risque polygénique et de la même échelle de niveaux de concentration de cholestérol dans les lipoprotéines de basse densité, la deuxième distribution statistique donnant en fonction de ces échelles une répartition des individus d'un deuxième sous-échantillon d'individus hypercholestérolémiques sévères ne présentant pas de mutation génétique dans l'ensemble de gènes;

## Description

### DOMAINE TECHNIQUE

Divers exemples de réalisation se rapportent généralement un procédé de détermination d'un profil hypercholestérolémique pour un patient atteint d'hypercholestérolémie sévère et un dispositif associé.

### ARRIERE PLAN TECHNIQUE

L'hypercholestérolémie héréditaire, dite hypercholestérolémie familiale (HF) d'origine monogénique (autosomique dominante/récessive) est liée à la présence de mutations rares chez les patients (« mutés ») et constitue actuellement la seule étiologie démontrée d'hypercholestérolémie sévère.

Il existe cependant une proportion notable de formes d'hypercholestérolémie sévère qui paraissent héréditaires mais qui ne présentent pas de mutation (« non-mutés », formes non-monogéniques).

Ceci conduit à envisager d'autres étiologies, notamment polygéniques. À ce jour, les prévalences de ces différentes formes ne sont toujours pas clairement évaluées.

Des stratégies d'identification des patients HF étaient initialement basées sur des critères clinico-biologiques tels que ceux du Dutch Lipid Clinic Network (DLCN) décrits pour la première fois en 1998.

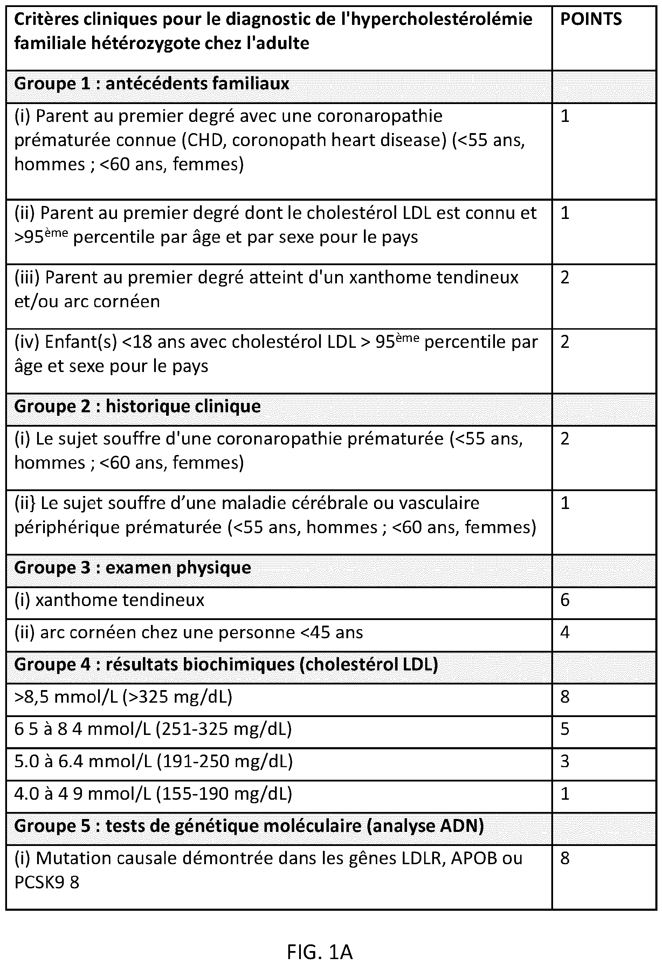

La Fig. 1A illustre une classification actuellement utilisée en France sur la base des critères DLCN pour le dépistage génétique de l'HF.

Selon cette méthode, la valeur du seuil de dépistage génétique de l'hypercholestérolémie sévère a été proposée, en l'absence de traitement, à 1,9 g.L⁻¹ de LDLc, et cette valeur définit aussi la limite inférieure de l'hypercholestérolémie sévère.

La concentration, notée ici LDLc, correspond à la concentration de cholestérol dans les lipoprotéines de basse densité (LDL cholestérol, LDLc), par exemple mesure dans un échantillon biologique, de préférence un échantillon de sang. Dans la présente par concentration circulante on entend la concentration mesurée dans un échantillon de sang.

Ce niveau de LDLc correspond au 90^{ème} percentile de la distribution des concentrations circulantes de LDLc dans la population générale adulte française. Dans la mesure où la prévalence de l'HF monogénique (individus « mutés ») est estimée à 1/300 (soit 0,33%), cela signifie qu'une majorité des hypercholestérolémies dites sévères (LDLc > 1,9 g.L⁻¹) ne sont pas d'origine monogénique.

Il est souhaitable d'améliorer la situation et d'apporter une stratégie de classification de patient prenant en compte différentes origines d'hypercholestérolémies sévères.

### RÉSUMÉ

L'étendue de la protection est définie par les revendications indépendantes. Les modes de réalisation, les exemples et les caractéristiques, le cas échéant, décrits dans la présente spécification qui ne relèvent pas de la protection doivent être interprétés comme des exemples utiles à la compréhension des divers modes de réalisation ou exemples qui relèvent de la protection.

Selon un premier aspect, un procédé in-vitro de détermination d'un profil hypercholestérolémique pour un patient hypercholestérolémique sévère est décrit. Le procédé est mis en oeuvre par ordinateur et comprenant :
- une obtention d'une première distribution statistique en fonction d'une échelle de niveaux de score de risque polygénique et d'une échelle de niveaux de concentration de cholestérol dans des lipoprotéines de basse densité, la première distribution statistique donnant en fonction de ces échelles une répartition des individus d'un premier sous-échantillon d'individus hypercholestérolémiques sévères présentant au moins une mutation génétique à l'origine d'une hypercholestérolémie pour au moins un gène parmi un ensemble de gènes;
- une obtention d'une deuxième distribution statistique en fonction de la même échelle de niveaux du score de risque polygénique et de la même échelle de niveaux de concentration de cholestérol dans les lipoprotéines de basse densité, la deuxième distribution statistique donnant en fonction de ces échelles une répartition des individus d'un deuxième sous-échantillon d'individus hypercholestérolémiques sévères ne présentant pas de mutation génétique dans l'ensemble de gènes;
- une obtention, par lecture dans une mémoire, de données déterminées à partir d'au moins un échantillon biologique pour le patient, les données comprenant :
   ∘ une information génétique sur la présence ou non d'au moins une mutation génétique à l'origine d'une hypercholestérolémie pour au moins un gène parmi l'ensemble de gènes;
   ∘ un score de risque polygénique ;
   ∘ une concentration de cholestérol dans les lipoprotéines de basse densité en absence de traitement;
- une détermination, pour le patient, d'une position dans une des distributions statistiques en fonction du score de risque polygénique et de la concentration de cholestérol dans les lipoprotéines de basse densité, la position étant déterminée dans la première distribution statistique lorsque l'information génétique obtenue pour le patient indique la présence d'au moins une mutation et dans la deuxième distribution lorsque l'information génétique obtenue pour le patient indique l'absence de mutation ;
- une détermination du profil hypercholestérolémique du patient en fonction de la position déterminée ;

l'échelle de niveaux de concentration de cholestérol dans les lipoprotéines de basse densité étant obtenue sur la base d'une distribution statistique des concentrations de cholestérol dans les lipoprotéines de basse densité obtenues pour les individus de la population totale constituée des premier et deuxième sous-échantillons d'individus;
l'échelle de niveaux de score de risque polygénique étant obtenue sur la base de la distribution statistique des scores de risque polygénique obtenus pour les individus du premier sous-échantillon d'individus.

Selon un ou plusieurs modes de réalisation, la détermination du profil hypercholestérolémique du patient comprend une détermination d'une classe d'appartenance du patient sur la base de la position obtenue, la classe étant
- soit une première classe lorsque l'information génétique indique la présence d'une mutation génétique;
- soit une deuxième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution statistique présentant une valeur supérieure ou égale à un premier seuil ;
- soit une troisième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution statistique indiquent une valeur supérieure ou égale à un deuxième seuil et inférieure strictement au premier seuil;
- soit une quatrième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution indiquent une valeur strictement inférieure au deuxième seuil.

Selon un ou plusieurs modes de réalisation, la première classe correspond à un profil hypercholestérolémique avec hérédité dite monogénique confirmée, dans lequel la deuxième classe correspond à un profil hypercholestérolémique avec hérédité dite polygénique, dans lequel la troisième classe correspond à un profil hypercholestérolémique avec hérédité dite indéterminée, dans lequel la quatrième classe correspond à un profil hypercholestérolémique avec hérédité dite monogénique de cause non identifiée.

Selon un ou plusieurs modes de réalisation, la première distribution statistique est obtenue par régression de type modèle additif généralisé à partir d'une distribution statistique initiale obtenue le premier sous-échantillon d'individus.

Selon un ou plusieurs modes de réalisation, la deuxième distribution statistique est obtenue par régression de type modèle additif généralisé à partir d'une distribution statistique initiale obtenue le deuxième sous-échantillon d'individus.

Selon un ou plusieurs modes de réalisation, l'échelle de niveaux de concentration de cholestérol dans les lipoprotéines de basse densité est une échelle en déciles dont les déciles sont calculés sur la base d'une distribution statistique des concentrations de cholestérol dans les lipoprotéines de basse densité obtenues à partir d'échantillon biologiques pour les individus de la population totale constituée des premier et deuxième sous-échantillons d'individus.

Selon un ou plusieurs modes de réalisation, l'échelle de niveaux de score de risque polygénique est une échelle en déciles (respectivement percentiles) calculés sur la base de la distribution statistique des scores de risque polygénique obtenus à partir d'échantillons biologiques pour les individus du premier sous-échantillon d'individus.

Selon un ou plusieurs modes de réalisation, les première et deuxième distributions statistiques donnent la répartition des individus en déciles (respectivement percentiles).

Selon un ou plusieurs modes de réalisation, le deuxième seuil correspond au 2^{ème} décile.

Selon un ou plusieurs modes de réalisation, l'ensemble des gènes comprend au moins : *LDLR, APOB, PCSK9, APOE.*

Selon un ou plusieurs modes de réalisation, la détermination du profil hypercholestérolémique du patient comprend : une prédiction d'un niveau de risque d'accident cardiovasculaire pour le patient sur la base de la position obtenue et d'une troisième distribution statistique fonction de la même échelle de niveaux de score de risque polygénique et de la même échelle de niveaux de concentration de cholestérol dans des lipoprotéines de basse densité, la troisième distribution statistique donnant en fonction de ces échelles un niveau de risque d'accident cardiovasculaire.

Selon un ou plusieurs modes de réalisation, le niveau de risque est calculé dans la troisième distribution statistique comme une valeur médiane du score CAC, Coronary Artery Calcium, de la distribution statistique des scores CAC obtenus pour le sous-ensemble d'individus ayant le même niveau de score de risque polygénique que le patient et le même niveau de concentration de cholestérol dans des lipoprotéines de basse densité, ce sous-ensemble d'individus étant soit un sous-ensemble du premier sous-échantillon d'individus lorsque l'information génétique obtenue pour le patient indique la présence d'au moins une mutation, soit un sous-ensemble du deuxième sous-échantillon d'individus lorsque l'information génétique obtenue pour le patient indique l'absence de mutation.

Selon un autre aspect, un dispositif comprend des moyens pour mettre en oeuvre un procédé selon le premier aspect.

Le dispositif peut comprendre des moyens logiciels et/ou matériels pour exécuter une ou plusieurs ou toutes les étapes du procédé selon le premier aspect. Ces moyens peuvent comprendre au moins un processeur et au moins une mémoire stockant des instructions qui, lorsqu'elles sont exécutées par au moins un processeur, amènent l'appareil à exécuter une ou plusieurs ou toutes les étapes du procédé selon le premier aspect. Ces moyens peuvent comprendre des circuits électroniques (par exemple, des circuits de traitement) pour exécuter une ou plusieurs ou toutes les étapes du procédé selon le premier aspect.

Selon un autre aspect, un appareil comprend au moins un processeur et au moins une mémoire stockant des instructions qui, lorsqu'elles sont exécutées par au moins un processeur, cause l'exécution par l'appareil une ou plusieurs ou toutes les étapes d'un procédé selon le premier aspect.

Selon un autre aspect, un programme informatique comprend des instructions qui, lorsqu'elles sont exécutées par un appareil, amènent l'appareil à exécuter effectuer une ou plusieurs ou toutes les étapes d'un procédé selon le premier aspect.

Selon un autre aspect, un support lisible par ordinateur non transitoire comprend des instructions de programme qui y sont stockées pour permettre à un appareil d'exécuter une ou plusieurs ou toutes les étapes d'un procédé selon le premier aspect.

### BRÈVE DESCRIPTION DES DESSINS

Les exemples de réalisation seront mieux compris à la lumière de la description détaillée ci-après et des dessins qui l'accompagnent, lesquels ne sont donnés qu'à titre d'illustration et ne sont donc pas limitatifs de la présente divulgation.
La Fig. 1A, déjà décrite, illustre une classification actuellement utilisée dans le monde selon les recommandations de l'OMS (WHO/HGN/FH/CONS/99.2) sur la base des critères DLCN pour le dépistage génétique de l'HF selon un exemple.
La Fig. 1B montre le risque de (CAD, pour Coronary Artery Disease) en fonction de la concentration LDLc selon un exemple.
La Fig. 2 montre une distribution statistique de valeurs de score polygénique (score PG) pour des individus « mutés » et des individus « non-mutés » selon un exemple.
La Fig. 3 représente la variation du nombre de prescription de dépistage de l'HF et du pourcentage de patients génétiquement confirmés HF.
La Fig. 4 montre répartition des patients « mutés » et « non-mutés » en fonction des valeurs de déciles de LDLc selon un exemple.
La Fig. 5 montre répartition des patients « mutés » et « non-mutés » en fonction des déciles de Score PG selon un exemple.
Les Fig. 6 à 11 représentent chacune des distributions statistiques obtenues pour un sous-échantillon de patients « mutés » et un sous-échantillon de patients « non-mutés » en fonction de leurs valeurs de LDLc et de Score PG selon un exemple.
La Fig. 12 illustre la méthode d'obtention des classes sur la base des distributions statistiques des figures 8 et 9.
La Fig. 13 montre un organigramme d'un procédé de classification selon un ou plusieurs exemples de réalisation.

Il convient de noter que ces dessins ont pour but d'illustrer divers aspects des dispositifs, procédés et structures utilisés dans les exemples de réalisation décrits ici. L'utilisation de numéros de référence similaires ou identiques dans les différentes figures a pour but d'indiquer la présence d'un élément ou d'une caractéristique similaire ou identique.

### DESCRIPTION DÉTAILLÉE

Des exemples détaillés de réalisations sont présentés ici. Toutefois, les détails structurels et/ou fonctionnels spécifiques divulgués ici sont simplement représentatifs aux fins de la description des exemples de réalisation et de la compréhension claire des principes sous-jacents. Toutefois, ces exemples de réalisation peuvent être mis en pratique sans ces détails spécifiques. Ces exemples de réalisation peuvent être concrétisés sous de nombreuses autres formes, avec diverses modifications, et ne doivent pas être interprétés comme se limitant aux seules réalisations exposées ici. En outre, les figures et les descriptions peuvent avoir été simplifiées pour illustrer des éléments et/ou des aspects pertinents pour une bonne compréhension de la présente invention, tout en éliminant, par souci de clarté, de nombreux autres éléments qui peuvent être bien connus dans l'art ou ne pas être pertinents pour la compréhension de l'invention.

Dans la présente, par « patient » ou « sujet », il est entendu tout individu susceptible d'avoir, par exemple de l'hypercholestérolémie, par exemple de l'hypercholestérolémie sévère, il peut s'agir par exemple d'un humain quel que soit son sexe et/ou âge. Il peut s'agir par exemple d'un humain, par exemple un adolescent, un adulte. Il peut s'agir par exemple d'un humain âgé de 12 ans à 99 ans. Il peut s'agir par exemple d'un adolescent avec un âge compris de 12 à 17 ans, un adulte avec un âge de 18 à 99 ans, de préférence un adulte avec un âge de 18 à 99 ans. Dans ce document, on utilisera indifféremment le terme « individu » ou « patient ».

Dans la présente, par hypercholestérolémie ou hyperlipidémie on entend une pathologie dans laquelle la concentration sanguine de lipides, par exemple de cholestérol, de triglycérides et/ou de phospholipides, est supérieure à une valeur de référence chez un sujet sain. Il peut s'agir par exemple d'une pathologie dans laquelle la concentration totale de cholestérol est supérieure à une valeur seuil de référence chez un sujet sain. Il peut s'agir par exemple d'hypercholestérolémie ou d'hyperlipidémie telle que mentionnée dans la référence bibliographique « Hyperlipidemia in coronary heart disease. II. Genetic analysis of lipid levels in 176 families and delineation of a new inherited disorder, combined hyperlipidemia" J L Goldstein, H G Schrott, W R Hazzard, E L Bierman, A G Motulsky J Clin Invest. 1973 Jul;52(7):1544-68. PMID: 4718953 ou "Familial Hypercholesterolemia". Levenson AE, de Ferranti SD. 2023 Nov 28. In: Feingold KR, Anawalt B, Blackman MR, Boyce A, Chrousos G, Corpas E, de Herder WW, Dhatariya K, Dungan K, Hofland J, Kalra S, Kaltsas G, Kapoor N, Koch C, Kopp P, Korbonits M, Kovacs CS, Kuohung W, Laferrère B, Levy M, McGee EA, McLachlan R, New M, Purnell J, Sahay R, Shah AS, Singer F, Sperling MA, Stratakis CA, Trence DL, Wilson DP, editors. Endotext [Internet]. South Dartmouth (MA): MDText.com, Inc.; 2000-. PMID: 27809433

Dans la présente, par « patient » ou « individu » hypercholestérolémique sévère on entend un individu, sujet ou patient, pour lequel, en l'absence de traitement de l'hypercholestérolémie, la concentration de cholestérol dans les lipoprotéines de basse densité (LDL cholestérol, LDLc), mesurée dans un échantillon biologique, est supérieure au seuil d'hypercholestérolémie sévère, fixé à 1,9 g.L⁻¹. Ce seuil correspond 90ème au percentile de la population française. C'est aussi le seuil d'FH Possible selon le DLCN (Dutch Lipid Clinic Network).

Dans la présente par concentration sanguine du cholestérol total ou concentration du cholestérol total dans un échantillon de sang, on entend la concentration sanguine du cholestérol total comprenant la somme de concentration de cholestérol dans les lipoprotéines de très basse densité (VLDL), basse densité (LDL) et dans les lipoprotéines de haute densité (HDL). Par exemple, la valeur seuil de référence de la concentration de cholestérol total est 200 mg.dL⁻¹. Par exemple, la valeur seuil de référence de la concentration cholestérol LDL est 130 mg.dL⁻¹. Par exemple, la valeur seuil de référence de la concentration cholestérol HDL est 40 mg.dL⁻¹. Par exemple, la valeur seuil de référence de la concentration triglycérides est 150 mg.dL⁻¹. Par exemple, les concentrations de référence de cholestérol total, cholestérol LDL, cholestérol HDL et de triglycérides sont de préférence : cholestérol total : inférieure à 200 mg.dL⁻¹, cholestérol LDL: inférieure à 130 mg.dL⁻¹, cholestérol HDL : supérieure à 40 mg.dL⁻¹, et triglycérides : inférieure à 150 mg.dL⁻¹

Dans la présente, la détermination de la concentration de cholestérol dans les lipoprotéines de basse densité (LDL) peut être réalisée dans un échantillon biologique par tout procédé adapté connu de la personne du métier. Il peut s'agir par exemple du procédé décrit dans le document « Methods for measurement of LDL-cholesterol: a critical assessment of direct measurement by homogeneous assays versus calculation. »Nauck M, Warnick GR, Rifai N. Clin Chem. 2002 Feb;48(2):236-54. PMID: 11805004.

Dans la présente, par « échantillon biologique» on entend tout liquide biologique, par exemple, il peut s'agir d'un échantillon de sang, de plasma, de sérum, de liquide synovial, de peau, etc. Il peut s'agir de préférence d'un échantillon de sang.

Dans la présente, la détermination de la concentration de cholestérol dans les lipoprotéines de basse densité (LDL) peut être réalisée à partir d'un échantillon biologique par tout procédé adapté connu de la personne du métier. Par exemple, la concentration de cholestérol dans les lipoprotéines de basse densité peut être mesurée selon toute méthode appropriée et peut être exprimée par exemple en mg.dL⁻¹, ou g.L⁻¹ ou en mmol.L⁻¹.

La concentration notée ici LDLc correspond à la concentration de cholestérol dans les lipoprotéines de basse densité (LDL cholestérol, LDLc).

Dans la présente par mutation génétique associée et/ou induisant une hypercholestérolémie, on entend toute mutation d'au moins un gène susceptible de modifier la concentration sanguine de lipides, par exemple de cholestérol et/ou de triglycérides d'un sujet ou patient. Il peut s'agir par exemple d'une mutation génétique d'au moins un gène parmi un ensemble de gènes. Dans la présente, le gène peut être tout gène connu de la personne du métier dans lequel au moins une mutation est susceptible de modifier la concentration sanguine de lipides. Dans la présente l'ensemble de gène peut comprendre au moins un gène choisi parmi les gènes *LDLR, APOB, PCSK9, APOE.* Il peut s'agir par exemple d'au moins une mutation dans au moins un gène choisi parmi *LDLR, APOB, PCSK9, APOE.*

Dans la présente par gène *LDLR* on entend le gène codant pour le récepteur des lipoprotéines de basse densité (*LDLR* « low density lipoprotein receptor » en anglais). Il s'agit du gène *LDLR* de séquence avec la référence NCBI NM_000527.5 (https://www.ncbi.nlm.nih.gov/nucleotide/NM_000527.5)

Dans la présente par mutation du gène *LDLR* on entend toute mutation connue de la personne du métier susceptible de modifier la structure et/ou l'activité biologique de récepteur des lipoprotéines de basse densité. Il peut s'agir d'au moins une mutation du gène *LDLR* tel que décrite dans « ClinVar database of global familial hypercholesterolemia-associated DNA variants ». Iacocca MA, Chora JR, Carrié A, Freiberger T, Leigh SE, Defesche JC, Kurtz CL, DiStefano MT, Santos RD, Humphries SE, Mata P, Jannes CE, Hooper AJ, Wilemon KA, Benlian P, O'Connor R, Garcia J, Wand H, Tichy L, Sijbrands EJ, Hegele RA, Bourbon M, Knowles JW; ClinGen FH Variant Curation Expert Panel. Hum Mutat. 2018 Nov;39(11):1631-1640. doi: 10.1002/humu.23634. PMID: 30311388; PMCID: PMC6206854 et/ou identifiée selon le procécé décrit dans "Standards and guidelines for the interprétation of sequence variants: a joint consensus recommendation of the American College of Medical Genetics and Genomics and the Association for Molecular Pathology." Richards S, Aziz N, Bale S, Bick D, Das S, Gastier-Foster J, Grody WW, Hegde M, Lyon E, Spector E, Voelkerding K, Rehm HL; ACMG Laboratory Quality Assurance Committee. Genet Med. 2015 May;17(5):405-24. doi: 10.1038/gim.2015.30. Epub 2015 Mar 5. PMID: 25741868; PMCID: PMC4544753 et/ou "The Clinical Genome Resource (ClinGen) Familial Hypercholesterolemia Variant Curation Expert Panel consensus guidelines for LDLR variant classification."Chora JR, lacocca MA, Tichý L, Wand H, Kurtz CL, Zimmermann H, Leon A, Williams M, Humphries SE, Hooper AJ, Trinder M, Brunham LR, Costa Pereira A, Jannes CE, Chen M, Chonis J, Wang J, Kim S, Johnston T, Soucek P, Kramarek M, Leigh SE, Carrié A, Sijbrands EJ, Hegele RA, Freiberger T, Knowles JW, Bourbon M; ClinGen Familial Hypercholesterolemia Expert Panel. Genet Med. 2022 Feb;24(2):293-306. PMID: 34906454. Il peut s'agir par exemple d'une mutation du gène LDLR telle que décrite dans la base de données Leiden Open Variation Database (LOVD) (https://databases.lovd.nl/shared/genes/ LDLR ). Il peut s'agir par exemple d'une mutation du gène LDLR telle que décrite dans la base de données Clingen (https://erepo.clinicalgenome.org/evrepo/ui/summary/classifications?columns=gene&value s=LDLR&matchTypes=exact&pgSize=25).

Dans la présente par gène APOB on entend le gène codant pour l'apolipoprotéine B. Il s'agit du gène APOB de séquence avec la référence NCBI NM_000384.3 (https://www.ncbi.nlm.nih.gov/nucleotide/NM_000384.3).

Dans la présente par mutation du gène *APOB* on entend toute mutation connue de la personne du métier susceptible de modifier la structure et/ou l'activité biologique de l'apolipoprotéine B. Il peut s'agir d'au moins une mutation du gène APOB tel que décrite dans « ClinVar database of global familial hypercholesterolemia-associated DNA variants ». Iacocca MA, Chora JR, Carrié A, Freiberger T, Leigh SE, Defesche JC, Kurtz CL, DiStefano MT, Santos RD, Humphries SE, Mata P, Jannes CE, Hooper AJ, Wilemon KA, Benlian P, O'Connor R, Garcia J, Wand H, Tichy L, Sijbrands EJ, Hegele RA, Bourbon M, Knowles JW; ClinGen FH Variant Curation Expert Panel. Hum Mutat. 2018 Nov;39(11):1631-1640. doi: 10.1002/humu.23634. PMID: 30311388; PMCID: PMC6206854 et/ou identifiée selon le procécé décrit dans "Standards and guidelines for the interprétation of sequence variants: a joint consensus recommendation of the American College of Medical Genetics and Genomics and the Association for Molecular Pathology." Richards S, Aziz N, Bale S, Bick D, Das S, Gastier-Foster J, Grody WW, Hegde M, Lyon E, Spector E, Voelkerding K, Rehm HL; ACMG Laboratory Quality Assurance Committee. Genet Med. 2015 May;17(5):405-24. doi: 10.1038/gim.2015.30. Epub 2015 Mar 5. PMID: 25741868; PMCID: PMC4544753. Il peut s'agir par exemple d'une mutation du gène APOB telle que décrite dans la base de données LOVD (https://databases.lovd.nl/shared/genes/APOB).

Dans la présente par gène *PCSK9* on entend le gène codant pour la proprotéine convertase subtilisin/kexin type 9. Il s'agit du gène PCSK9 de séquence avec la référence NCBI NM_174936.4 (https://www.ncbi.nlm.nih.gov/nucleotide/NM_174936.4)

Dans la présente par mutation du gène *PCSK9* on entend toute mutation connue de la personne du métier susceptible de modifier la structure et/ou l'activité biologique de la proprotéine convertase subtilisin/kexin type 9.

Il peut s'agir d'au moins une mutation du gène PCSK9 tel que décrite dans « ClinVar database of global familial hypercholesterolemia-associated DNA variants ». Iacocca MA, Chora JR, Carrié A, Freiberger T, Leigh SE, Defesche JC, Kurtz CL, DiStefano MT, Santos RD, Humphries SE, Mata P, Jannes CE, Hooper AJ, Wilemon KA, Benlian P, O'Connor R, Garcia J, Wand H, Tichy L, Sijbrands EJ, Hegele RA, Bourbon M, Knowles JW; ClinGen FH Variant Curation Expert Panel. Hum Mutat. 2018 Nov;39(11):1631-1640. doi: 10.1002/humu.23634. PMID: 30311388; PMCID: PMC6206854 et/ou identifiée selon le procécé décrit dans "Standards and guidelines for the interprétation of sequence variants: a joint consensus recommendation of the American College of Medical Genetics and Genomics and the Association for Molecular Pathology." Richards S, Aziz N, Bale S, Bick D, Das S, Gastier-Foster J, Grody WW, Hegde M, Lyon E, Spector E, Voelkerding K, Rehm HL; ACMG Laboratory Quality Assurance Committee. Genet Med. 2015 May;17(5):405-24. doi: 10.1038/gim.2015.30. Epub 2015 Mar 5. PMID: 25741868; PMCID: PMC4544753. Il peut s'agir par exemple d'une mutation du gène PCSK9 telle que décrite dans la base de données LOVD(https://databases.lovd.nl/shared/genes/PCSK9 ou « Mutations and polymorphisms in the proprotein convertase subtilisin kexin 9 (PCSK9) gène in cholestérol metabolism and disease », Abifadel M, Rabès JP, Devillers M, Munnich A, Erlich D, Junien C, Varret M, Boileau C.Hum Mutat. 2009 Apr;30(4):520-9. doi: 10.1002/humu.20882. PMID: 19191301.

Dans la présente par gène *APOE* on entend le gène codant pour l'apolipoprotéine E. Il s'agit du gène *APOE* de séquence avec la référence NCBI NM_000041.4 https://www.ncbi.nlm.nih.gov/nucleotide/NM_000041.4)

Dans la présente par mutation du gène *APOE* on entend toute mutation connue de la personne du métier susceptible de modifier la structure et/ou l'activité biologique de l'apolipoprotéine E. Il peut s'agir d'au moins une mutation du gène *APOE* tel que décrite dans « ClinVar database of global familial hypercholesterolemia-associated DNA variants ». Iacocca MA, Chora JR, Carrié A, Freiberger T, Leigh SE, Defesche JC, Kurtz CL, DiStefano MT, Santos RD, Humphries SE, Mata P, Jannes CE, Hooper AJ, Wilemon KA, Benlian P, O'Connor R, Garcia J, Wand H, Tichy L, Sijbrands EJ, Hegele RA, Bourbon M, Knowles JW; ClinGen FH Variant Curation Expert Panel. Hum Mutat. 2018 Nov;39(11):1631-1640. doi: 10.1002/humu.23634. PMID: 30311388; PMCID: PMC6206854 et/ou identifiée selon le procécé décrit dans "Standards and guidelines for the interprétation of sequence variants: a joint consensus recommendation of the American College of Medical Genetics and Genomics and the Association for Molecular Pathology." Richards S, Aziz N, Bale S, Bick D, Das S, Gastier-Foster J, Grody WW, Hegde M, Lyon E, Spector E, Voelkerding K, Rehm HL; ACMG Laboratory Quality Assurance Committee. Genet Med. 2015 May;17(5):405-24. doi: 10.1038/gim.2015.30. Epub 2015 Mar 5. PMID: 25741868; PMCID: PMC4544753. Il peut s'agir par exemple d'une mutation du gène APOE telle que décrite dans la base de données LOVD https://databases.lovd.nl/shared/genes/APOE.

Dans la présente, l'obtention de l'information génétique sur la présence d'une mutation génétique peut être réalisée par tout procédé adapté connue de la personne du métier. Il peut s'agir par exemple d'un procédé comprenant à partir d'un échantillon biologique, par exemple un échantillon de sang, une détermination et/ou détection d'une mutation dans un gène connu de la personne du métier. Il peut s'agir par exemple d'un procédé choisi parmi le polymorphisme de longueur des fragments de restriction (RFLP), le polymorphisme de conformation des simples brins (SSCP), l'électrophorèse sur gel en gradient dénaturant (DGGE), une réaction en chaîne par polymérase (PCR) et séquençage de Sanger, une analyse des courbes de fusion à haute résolution (HRM), un séquençage de nouvelle génération (NGS) également désigné par séquençage à haut débit ou séquençage massif parallèle, et/ou un séquençage « long read » ou la technique de MLPA (Multiplex Ligation dépendant Probe Amplification) qui permet de mettre en évidence les grands réarrangements. Il peut s'agir par exemple d'un séquençage à haut débit tel que décrit dans « An Overview of DNA Analytical Methods ». Arboleda VA, Xian RR. Methods Mol Biol. 2019;1897:385-402. doi: 10.1007/978-1-4939-8935-5_31. PMID: 30539459

Dans le cadre de ce document, on parlera de :
- patient ou individu « muté » pour signifier qu'au moins une mutation génétique d'au moins un gène à l'origine d'une hypercholestérolémie a été identifiée par une analyse génétique (par exemple par une technologie de séquençage);
- patient ou individu « non muté » pour signifier qu'aucune une mutation génétique à l'origine d'une hypercholestérolémie n'a été identifiée par une analyse génétique ;

En outre, on parlera d'hypercholestérolémie monogénique pour un patient « muté » atteint d'hypercholestérolémie, quel que soit le nombre de gène(s) muté(s) et/ou le nombre de mutations observées.

Un ou plusieurs exemples de réalisation concernent un procédé de classification des patients atteints d'hypercholestérolémie sévère et un dispositif associé.

Le procédé de classification est basé sur une analyse statistique effectuée à partir d'une population d'individus présentant une hypercholestérolémie sévère.

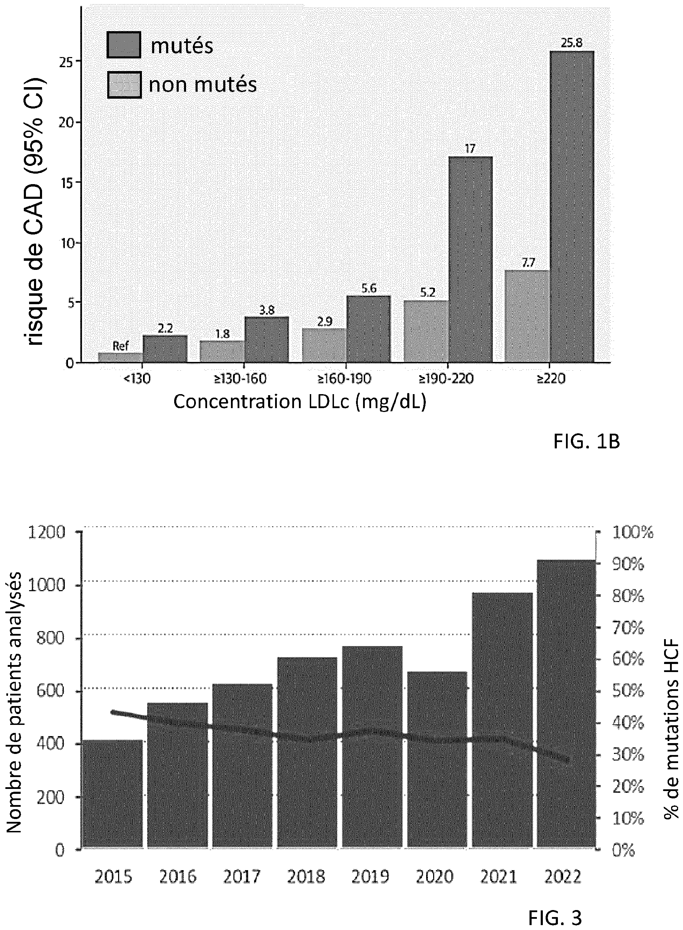

L'HF monogénique est associée à un risque élevé de coronaropathies (CAD, pour Coronary Artery Disease). En effet, il a été montré que ce risque était plus élevé chez les patients «mutés » par rapport aux patients «non-mutés», comme représenté sur la Fig. 1B.

La Fig. 1B compare le risque de CAD en fonction de la concentration en LDLc chez des patients «mutés » par rapport aux patients «non-mutés» pour une gamme de concentrations LDLc.

Sur la Fig. 1B, on constate que, pour des concentrations supérieures au seuil de dépistage génétique, le risque de CAD est beaucoup plus élevé, chez les patients «mutés » par rapport aux patients «non-mutés», avec un facteur multiplicatif supérieur à 3.

Les stratégies d'identification des patients HF basées sur des critères clinico-biologiques tels que ceux du Dutch Lipid Clinic Network (DLCN) ont été mis en place et présentent les limitations évoquées en introduction par référence à la Fig. 1A.

La valeur du seuil de dépistage génétique de l'hypercholestérolémie sévère est actuellement fixée, en l'absence de traitement, à 1,9 g.L⁻¹ de LDLc dans un échantillon de sang. Ce niveau de LDLc correspond également au 90^{ème} percentile de la distribution des concentrations sériques de LDLc dans la population générale française. Dans la mesure où la prévalence de l'HF monogénique (individus «mutés») est estimée à 1/300 (soit 0,33%), cela signifie qu'une majorité des hypercholestérolémies dites sévères (LDLc > 1,9 g.L⁻¹) ne sont pas d'origine monogénique.

Une partie des hypercholestérolémies sévères devrait donc correspondre à des formes polygéniques comme l'ont suggéré Talmud et al. dans [01], en proposant le calcul d'un score polygénique (Score PG). Ce score prend en compte une combinatoire de variants génétiques. Par exemple le score PG peut avantageusement permettre de déterminer, en fonction de la valeur du score obtenu, l'origine de la variation génétique, par exemple monogénique ou polygénique.

Dans la présente par score polygénique (Score PG), également désigné score de risque polygénique (ou PRS pour Polygenic Risk Score), on entend par exemple une valeur numérique dérivée d'une analyse génomique. Il peut être utilisé pour prédire la probabilité qu'un individu développe une certaine maladie ou caractéristique en fonction de son profil génétique. Il peut s'agir du score PG tel que décrit dans [01].

Dans la présente, l'obtention d'un score polygénique (Score PG) également dénommé score de risque polygénique, peut être réalisée par tout procédé adapté connu de la personne du métier. Le score polygénique peut être obtenu à partir de tout échantillon biologique connu de la personne du métier permettant d'analyser l'ADN génomique. Il peut s'agir par exemple d'un échantillon de sang, salive, phanères, cheveux, de peau et/ou cutané. Le score polygénique (Score PG) peut être obtenu/déterminé par exemple par séquençage de l'ADN génomique, par exemple selon le procédé tel que décrit dans [01].

Le score PG peut être utilisé pour prédire la probabilité qu'un individu développe une hypercholestérolémie, ce score permet avantageusement de déterminer un risque d'hypercholestérolémie, par exemple polygénique.

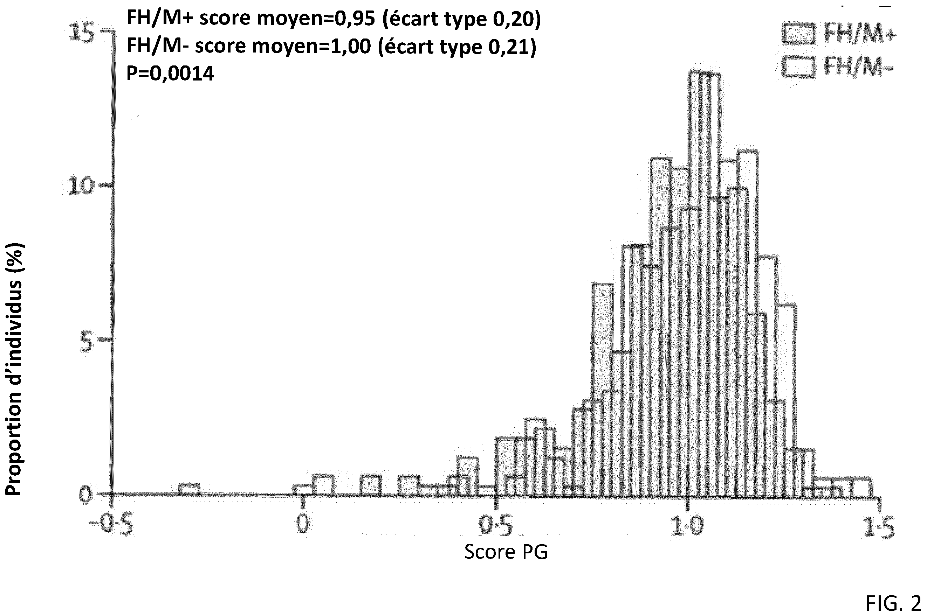

Le caractère discriminant du score PG est cependant difficile à préciser comme en atteste le chevauchement des courbes de distribution des valeurs de ce score entre individus «mutés» et «non-mutés» représenté à la Fig. 2.

La Fig. 2 montre la distribution statistique des valeurs de Score PG d'individus hypercholestérolémiques sévères suspectés d'hypercholestérolémie familiale (HF), d'une part pour les individus «mutés» (HF/M+) et d'autre part pour les individus «non-mutés» (HF/M-).

Le diagnostic moléculaire de l'HF peut être effectué au moyen de nouvelles technologies de séquençage (NGS). Il est ainsi possible d'identifier les mutations rares à l'origine des formes monogéniques (autosomique dominante/récessive) mais également de calculer le Score PG afin d'évaluer la part de la composante polygénique de ces hypercholestérolémies sévères.

Par ailleurs, une forte augmentation d'année en année du nombre de prescriptions pour hypercholestérolémie sévère a été observée alors qu'une baisse régulière du pourcentage d'HF confirmées par l'identification de mutation se produit sur la même période.

Ce phénomène est illustré par la Fig. 3 qui représente le nombre de prescription de dépistage de l'HF (échelle de gauche, barres verticales) et le pourcentage de patients (courbe) génétiquement confirmés HF par identification de mutations rares (échelle de droite).

Ces tendances opposées s'expliquent en grande partie par l'augmentation de la proportion des formes non-monogéniques et non héréditaires parmi les demandes croissantes d'analyse qui sont adressées aux structures réalisant le diagnostic génétique d'HF. Ceci est probablement lié à une certaine inadaptation des critères utilisés.

En effet, ces critères clinico-biologiques, issus de la classification DLCN, ont été décrits il y a 25 ans (1998), alors que le diagnostic génétique était peu développé et que le dépistage de l'HF était réalisé au sein de centres experts et par quelques spécialistes.

Or ce dépistage est maintenant proposé par de nombreuses structures plus diversifiées, en particulier pour répondre à une augmentation de la demande qui est justifiée par la nécessité d'une preuve d'HF monogénique pour pouvoir accéder à certaines thérapeutiques hypocholestérolémiantes innovantes et couteuses.

Quoiqu'il en soit, un des effets délétères observé est l'accroissement du nombre de rendus de résultat génétique négatif qui ne contribuent en rien à la prise en charge des patients. Ces résultats négatifs génèrent au contraire un sentiment d'échec de ce diagnostic sans pour autant éliminer définitivement la part d'une composante héréditaire dans l'hypercholestérolémie sévère de ces patients.

En l'état actuel, nous ne disposons d'aucune donnée moléculaire sur les hypercholestérolémies sévères en France. En conséquence, il apparaît qu'il n'est pas possible d'identifier clairement les différentes formes d'hérédités (monogéniques versus polygéniques) et les formes non héréditaires, ni de mesurer leur éventuelle différence de distribution en fonction des valeurs de LDLc ou encore leur niveau de risque cardiovasculaire respectif.

En réponse à ces manques, une analyse de données moléculaires d'une cohorte de patients hypercholestérolémiques sévères a été effectuée et a permis de générer la distribution conjointe des concentrations LDLc sans traitement, d'une part, et des scores PG, d'autre part pour cette cohorte d'individus. Sur cette base, il est possible de
- déterminer une plage de valeurs de LDLc sans traitement dans une population d'individus hypercholestérolémiques sévères comprenant à la fois des individus «mutés» et «non-mutés» afin de déterminer les niveaux (par exemple les déciles) de LDLc sans traitement permettant de préciser le pourcentage d'individus «mutés» et «non-mutés» en fonction de ces déciles, d'une part ;
- déterminer une plage de valeurs Score PG dans le sous-échantillon comprenant uniquement les individus hypercholestérolémiques sévères «mutés» afin de définir de les niveaux (par exemple les déciles) de Score PG permettant de décrire la distribution des patients «non-mutés», d'autre part.

À partir de ces valeurs (déciles de LDLc sans traitement et de Score PG dans une cohorte d'individus hypercholestérolémiques sévères), la distribution statistique conjointe des concentrations LDLc sans traitement et de scores PG des individus «mutés» et «non-mutés» a été générée et analysée. Sur la base de cette nouvelle distribution statistique, une méthode de classification des patients est introduite, qui est basée sur leur hérédité, qu'elle soit monogénique, polygénique, ou encore indéterminée.

La méthode de classification des patients permet :
- d'identifier les caractéristiques clinico-biologiques les plus distinctives de ces formes d'hérédité afin d'améliorer la sélection des patients candidats au dépistage génétique de l'HF et de proposer une redéfinition des critères de type « DLCN » dans la population d'hypercholestérolémies sévères.
- de modifier les modalités de rendu de résultat en précisant plus clairement la probable origine polygénique de l'HF ;
- d'étudier le risque cardiovasculaire respectif de ces différentes formes, ce qui permet d'améliorer leur prise en charge pour prévenir la survenue d'événements cardiovasculaires.

### Population étudiée et méthodes

Depuis 2015, 4358 patients (niveau "HF possible" selon DLCN correspondant à une valeur de LDLc maximum sans traitement ≥19 g.L⁻¹) ont été analysés en routine par technique NGS (ADH MASTR v2, Agilent/Multiplicom NV) permettant (i) d'identifier des mutations dans les régions codantes du LDLR, PCSK9, APOE, et du domaine de liaison de l'APOB et (ii) de calculer un score polygénique (Score PG) fondé sur 12 SNPs (Single Nucleotide Polymorphism) (GS12 ; cf. [01]).

Parmi cette population initiale de 4358 patients, nous avons étudié une sélection de 3188 cas index et apparentés pour lesquels nous disposions des bilans lipidiques sans traitement et qui répondaient aux critères de sélection suivant : LDLc maximum sans traitement ≥ 1,9 g.L⁻¹, triglycérides <4 g.L⁻¹et âge ≥ 18 ans.

Ces 3188 patients, représentatifs de la population hypercholestérolémique sévère, ont été soumis au dépistage de l'HF dans une structure réalisant le diagnostic génétique en France et les résultats obtenus étaient les suivants :
- 1574 ne présentaient aucune anomalie moléculaire («non-mutés»).
- 1407 présentaient une anomalie moléculaire («mutés»), dont
   ∘ 1275 dans le gène LDLR
   ∘ 97 dans le gène APOB
   ∘ 26 dans le gène PCSK9
   ∘ 9 dans le gène APOE
- 207 présentaient une anomalie moléculaire de signification inconnue qui ne permettait pas la prise en compte et n'ont pas été considérés dans la génération de la distribution statistique conjointe.

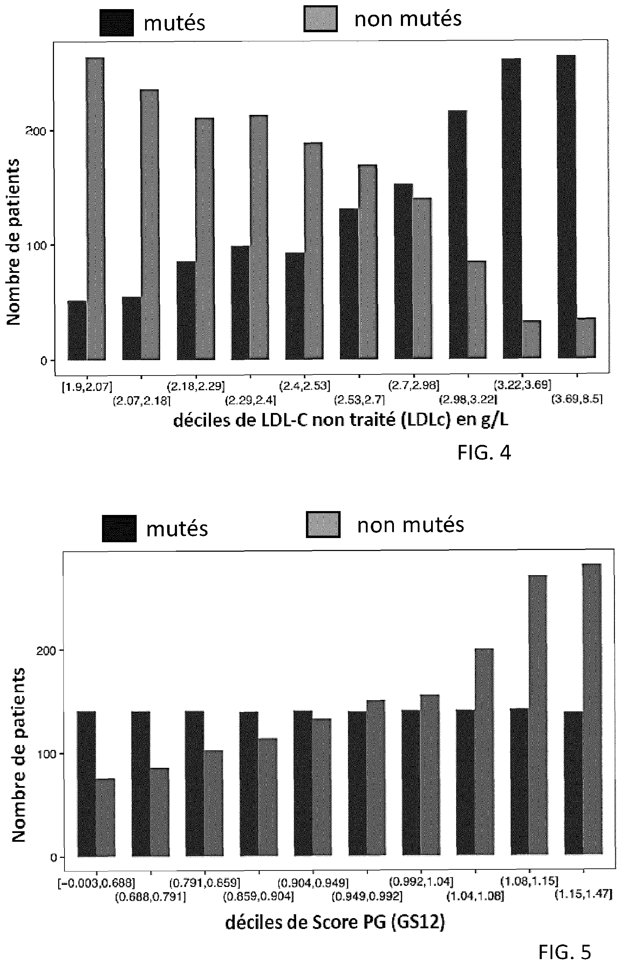

Au total, les résultats présentés dans les figures 4 à 11 correspondent à l'analyse d'un sous-échantillon de 1407 individus «mutés» et d'un sous-échantillon de 1574 individus «non-mutés», la cohorte étudiée étant au final constituée uniquement de ces deux sous-échantillons.

Pour 1355 d'entre eux (667 «mutés» et 688 «non-mutés»), nous disposions de leur valeur de CAC score (Coronary Artery Calcium) qui reflète l'atteinte de leurs artères coronaires et leur risque de CAD ; pour 2022 (1061 «mutés» et 961 «non-mutés») nous avions accès à leurs bilans lipidiques sous traitement.

Les proportions d'individus «mutés» et « non mutés » ont été estimées par décile de LDLc, de Score PG et par croisement (distribution jointe) de ces deux variables ordinales. Un modèle statistique a été développé pour analyser la densité de patients «mutés» et de patients «non-mutés» selon la distribution jointe de LDLc et de Score PG.

Pour cela, l'ensemble de l'échantillon a été découpé en sous-ensembles d'individus selon : (i) les quantiles (par exemple, les déciles) de concentration LDLc (sur la base de la distribution des valeurs de concentration LDLc sur l'ensemble de la cohorte étudiée, constituée d'un premier sous-échantillon d'individus « mutés » et d'un deuxième sous-échantillon d'individus « non-mutés ») et (ii) les quantiles (par exemple les déciles) de Score PG (sur la base de la distribution du score PG dans le premier sous-échantillon d'individus «mutés» uniquement).

Ainsi, les quantiles de concentration LDLc ont été déterminées sur l'ensemble de la cohorte étudiée, en découpant la plage de valeurs de concentration LDLc en N niveaux (ou N sous-plages de valeurs), chaque niveau correspondant à un quantile, par exemple à un décile si N=10, soit 100/N=10% de la cohorte constituée du sous-échantillon d'individus « mutés » et du sous-échantillon d'individus « non-mutés ». D'autres valeurs sont utilisables pour le nombre N. Le nombre N peut par exemple être choisi égal à 12, 15, 20 ou 100. Pour N=100, la plage de valeurs sera découpée en percentiles.

De manière connue, les quantiles d'une distribution statistique F fonction d'une variable X correspondent à une suite de N valeurs de X qui permettent de diviser en N (>=2) parts égales la population (i.e. la surface sous la courbe F) pour laquelle cette distribution est établie et définissent ainsi des sous-plages de valeurs. Ces sous-plages sont appelées également déciles.

Les quantiles de score PG ont été déterminées au sein du sous-échantillon d'individus « mutés » uniquement, en découpant cette plage de valeurs de score PG en N niveaux (ou sous-plages de valeurs), chaque niveau correspondant à un quantile, par exemple à un décile si N=10, soit 100/N=10% du sous-échantillon d'individus « mutés ». Cette même échelle a été appliquée au sous-échantillon d'individus « non-mutés ». D'autres valeurs sont utilisables pour le nombre N. Le nombre N peut par exemple être choisi égal à 12, 15, 20 ou 100. Pour N=100, la plage de valeurs sera découpée en percentiles.

Dans les exemples de réalisation décrits dans ce document, on suppose, pour simplifier l'exposé, que le nombre N est choisi égal à 10 pour l'échelle des concentrations LDLc et scores PG, ce qui aboutit à des échelles en déciles. Mais ces exemples sont généralisables à un nombre quelconque de niveaux.

La distribution statistique conjointe a été générée pour le sous-échantillon d'individus « mutés » sur la base des déciles de concentration LDLc et des déciles de score PG, chaque valeur de cette distribution statistique conjointe correspondant au nombre d'individus « mutés » pour un couple de valeurs composé d'un décile de concentration LDLc et d'un décile de score PG, selon les échelles de déciles établies comme indiqué ci-dessus.

De même, une autre distribution statistique conjointe a été générée pour le sous-échantillon d'individus « non-mutés » sur la base des déciles de concentration LDLc et des décile de score PG, chaque valeur de cette distribution statistique conjointe correspondant au nombre d'individus « non-mutés » pour un couple de valeurs composé d'un décile de concentration LDLc et d'un décile de score PG, selon les échelles de déciles établies comme indiqué ci-dessus, de sorte que les deux distributions statistiques conjointes pour le sous-échantillon d'individus « mutés » et le sous-échantillon d'individus « non-mutés » utilisent les mêmes échelles de déciles pour la concentration LDLc et le score PG.

Chacune des deux distributions statistiques conjointes a été normalisée par rapport au nombre total d'individus dans l'échantillon concerné. Ainsi la distribution statistique conjointe du sous-échantillon d'individus « mutés » a été rapportée au nombre total d'individus dans ce sous-échantillon, et de même pour le sous-échantillon d'individus « non-mutés ». Après normalisation, chaque point de la distribution statistique conjointe fait correspondre une proportion d'individus « mutés » et une proportion d'individus « non-mutés » (ces proportions pouvant s'exprimer en pourcentage ou en décile ou en percentile) à un couple de valeurs composé d'un décile de concentration LDLc et d'un décile de score PG.

Les proportions obtenues pour les distributions statistiques conjointes après normalisation ont été modélisées en fonction des déciles de concentration LDLc et de score PG par une méthode de régression de type modèle additif généralisé (en anglais, « generalized additive model », GAM), afin d'obtenir un lissage non linéaire des proportions calculées et donc des distributions statistiques conjointes après normalisation. Les proportions prédites par ce modèle pour chaque couple de décile de concentration LDLc et de décile de score PG ont été ordonnées par ordre croissant et leur distribution cumulée a été calculée, permettant ainsi de présenter, au moyen de distributions statistiques lissées (appelées par la suite «distributions cumulées »), un gradient de valeurs couplées de décile de concentration LDLc et de décile de score PG recouvrant une proportion croissante d'individus appartenant aux échantillons « muté » et « non-muté », respectivement.

Chacune des deux distributions cumulées ainsi obtenues constitue une distribution statistique de référence. Ces distributions statistiques permettent d'estimer la fréquence attendue d'individus « mutés » et d'individus « non mutés », respectivement, correspondant à un ensemble de couples de déciles de LDLc et de score PG.

### Résultats

Les résultats obtenus par la méthode sont décrits plus en détail au regard des figures 4 à 11. Sur les figures et dans le texte, on note « Dec » pour décile.

### 1^{ère} étape : définition des déciles de LDLc dans la cohorte de patients hypercholestérolémiques sévères

La Fig. 4 montre répartition des patients «mutés» et «non-mutés» en fonction des valeurs de déciles de LDLc.

L'échelle des valeurs de concentration de LDLc est comprise ici (Fig. 4) entre 1,9 et 8.5 et découpée en 10 déciles, chaque décile correspondant à 10% de l'ensemble de l'échantillon étudié, constitué, pour rappel, du sous-échantillon d'individus « mutés » et du sous-échantillon d'individus « non-mutés ». Il est à noter qu'avec une répartition en quantité (notamment décile), les sous-plages de valeurs correspondant aux déciles peuvent être de longueur variable. Par exemple, la première sous-plage correspondant au premier décile est entre 1,9 et 2,07, soit une sous-plage d'étendue 2,07-1,9=0,17 ; la deuxième sous-plage correspondant au deuxième décile est entre 2,07 et 2,18, soit une sous-plage d'étendue 2,18-2,07=0,11 ;

Dans cette distribution, on observe que la proportion de patients «non-mutés» diminue à mesure que la concentration en LDLc augmente, alors que celle des patients «mutés» augmente.

### 2^{ème} étape : définition des déciles de score polygénique (Score PG) dans la cohorte de patients hypercholestérolémiques sévères.

Cette étape prend comme référence uniquement les individus « mutés » (appelés ici aussi « monogéniques »). En effet, chez ces patients, on peut considérer que leur valeur de LDLc est principalement déterminée par la présence d'une mutation à effet fonctionnel majeur et non par une origine polygénique prépondérante.

La Fig. 5 montre répartition des patients « mutés » et « non-mutés » en fonction des déciles de Score PG.

L'échelle des valeurs de score PG est comprise ici entre -0,003 et 1,47 et découpée en 10 déciles, chaque décile correspondant à 10% du sous-échantillon d'individus « mutés ».

La définition de ces déciles permet de montrer une augmentation de la proportion de patients «non-mutés» avec l'augmentation du score PG.

### 3^{ème} étape : distributions statistiques des individus selon les déciles de LDLc et de Score PG, au sein des sous-échantillons de patients « mutés » d'une part et « non-mutés » d'autre part.

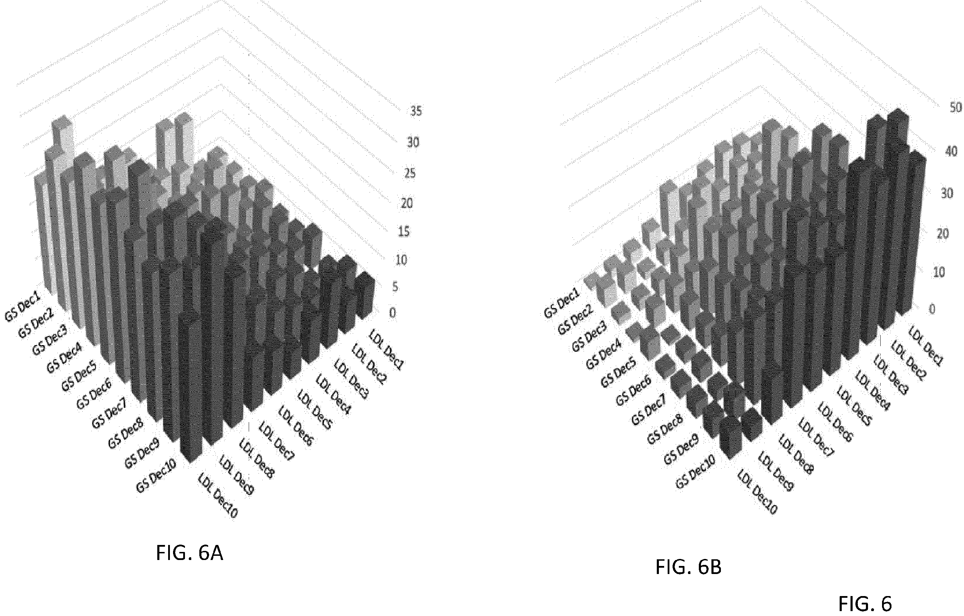

La Fig. 6 représente la distribution statistique conjointe, en nombre d'individus, pour le sous-échantillon des patients « mutés » (Fig. 6A) d'une part, et le sous-échantillon des patients « non-mutés » (Fig. 6B) d'autre part, en fonction de leurs valeurs de LDLc et de Score PG.

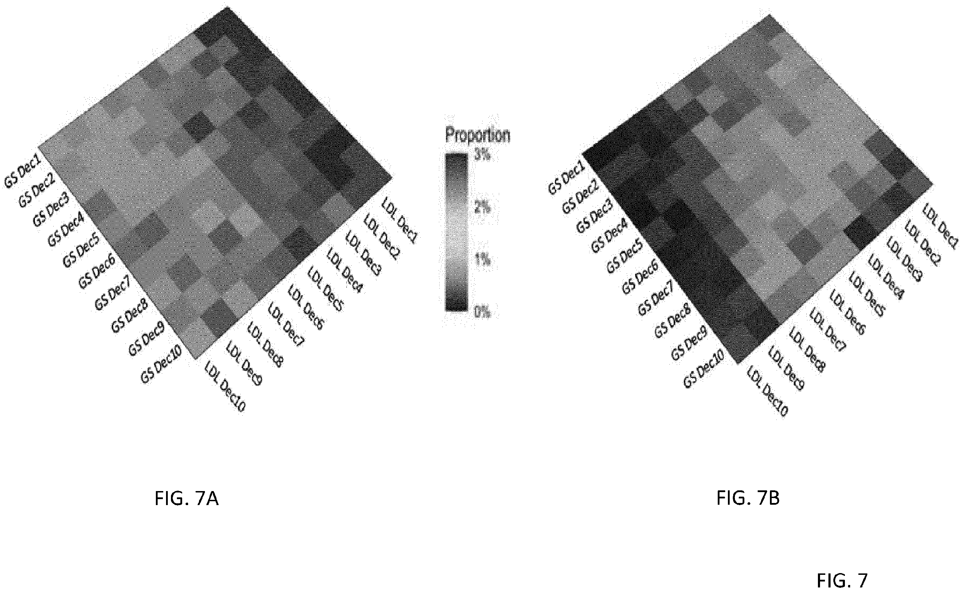

La Fig. 7 représente la distribution statistique conjointe normalisée, en proportion du sous-échantillon de patients « mutés », ou, respectivement, en proportion du sous-échantillon de patients « non-mutés », du sous-échantillon des patients « mutés » (Fig. 7A) et du sous-échantillon des patients « non-mutés » (FIG. 7B) en fonction de leurs valeurs de LDLc et de Score PG. Dans la Fig. 7, l'échelle verticale donnant les proportions de la population est exprimée en pourcentage, les valeurs s'échelonnant entre 0 et 3% du sous-échantillon considéré.

Pour les patients «mutés» (Fig. 7A) la répartition est essentiellement fonction de la valeur de LDLc (LDL Dec 1 vers LDL Dec 10). On observe cependant un effet du Score PG dans ce sous-échantillon. En effet, les patients du décile le plus élevé (GS Dec 10) se distribuent avec une plus forte proportion dans les trois déciles les plus élevés de LDLc (LDL Dec 8, 9 et 10).

Pour les patients «non-mutés» (FIG. 7B) le biais de répartition montre clairement un enrichissement de la proportion de patients en fonction des valeurs décroissantes de LDLc (LDL Dec 10 vers LDL Dec 1) et des valeurs croissantes de Score PG (GS Dec 1 vers GS Dec 10).

### 4^{ème} étape : prédiction de la distribution cumulée des individus selon les déciles de LDLc et de Score PG (modèle statistique de régression) au sein des sous-échantillons de patients «mutés» ou « non mutés ».

A partir de chacune des distributions statistiques de la Fig. 7, est prédite, sur la base d'un modèle statistique prédictif, une distribution cumulée, servant de distribution statistique de référence. Cette distribution peut être obtenue par régression, comme décrit dans ce document.

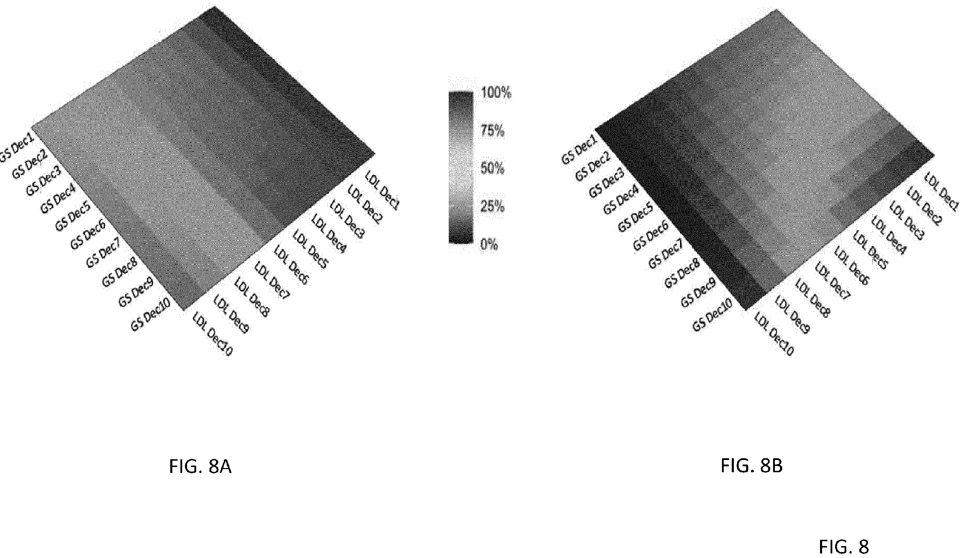

La Fig. 8 représente la distribution cumulée (en percentiles) obtenue pour la distribution des patients « mutés » (Fig. 8A) et obtenue pour la distribution des patients «non-mutés» (Fig. 8B) en fonction des valeurs de LDLc et de Score PG.

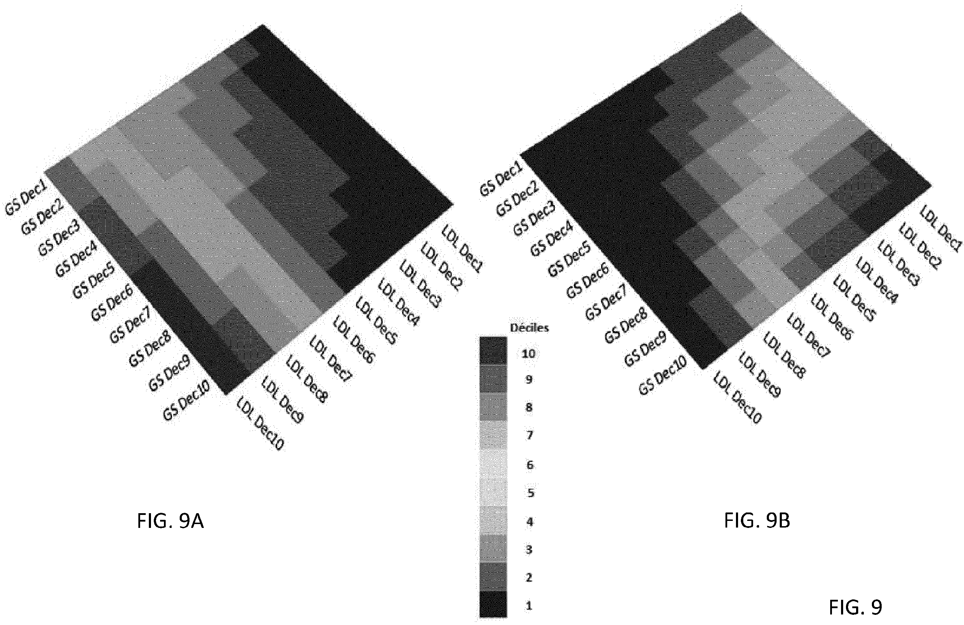

Dans la Fig. 8, l'échelle de couleurs donnant les proportions de la population est exprimée en percentiles, les valeurs s'échelonnant entre 0 et 100% du sous-échantillon considéré. La Fig. 9 représente la distribution cumulée (en déciles) obtenue pour la distribution des patients « mutés » (Fig. 9A) et obtenue pour la distribution des «non-mutés» (Fig. 9B) en fonction des valeurs de LDLc et de Score PG.

Dans la Fig. 9, l'échelle de couleurs donnant les proportions de la population est exprimée en déciles, les valeurs s'échelonnant entre 1 et 10 déciles par rapport au sous-échantillon considéré.

Dans cette distribution en déciles, on visualise clairement un biais de distribution des individus « non-mutés » (Fig. 9B). En effet, il existe une surreprésentation des patients des 5 déciles les plus élevés (6, Jaune à 10, rouge foncé), soit 50% des individus de ce sous-échantillon, dans 26% de la distribution (26 carrés sur 100).

### 5^{ème} étape : évaluation du risque cardiovasculaire et de la variation du LDLc au cours du suivi du patient

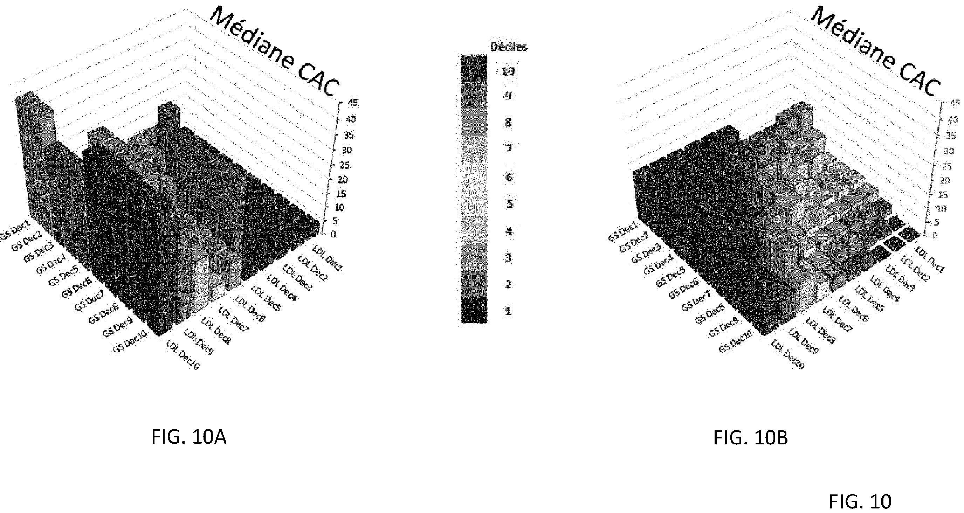

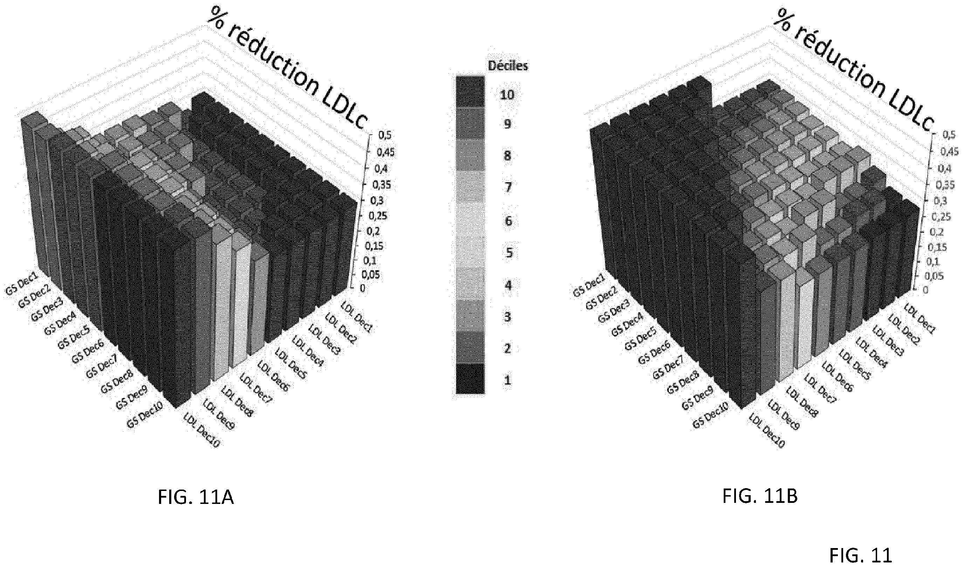

L'évaluation du risque cardiovasculaire (voir Fig. 10) et de la variation du LDLc au cours du suivi (voir Fig. 11) peut être effectuée pour des patients hypercholestérolémiques sévères à partir du modèle prédictif des Figs. 9A et 9B des distributions de patients « mutés » ou « non mutés » en fonction des combinaisons de LDLc et de Score PG donnés.

La Fig. 10 représente, selon l'axe vertical, pour chaque couple de valeurs de LDLc et de Score PG, la valeur médiane de la distribution statistique des valeurs du score CAC score (Coronary Artery Calcium) obtenues pour la catégorie de patients correspondant à ce couple de valeurs de LDLc et de Score PG.

Cette distribution est obtenue en fonction des déciles de la prédiction de distribution des patients « mutés » (Fig. 10A, avec n = 667 individus) et « non-mutés » (Fig. 10B, avec n = 688 individus) selon les valeurs de LDLc et de Score PG.

Dans la présente par « score calcique des artères coronaires » (« coronary artery calcium score » en anglais) on entend un score obtenu par des données d'imagerie coronarienne qui évalue la quantité de calcium dans les parois des vaisseaux sanguins du coeur. Ce score peut être mesuré par tomodensitométrie non invasive du coeur, avantageusement sans injection de produit de contraste. La quantité de calcium est indiquée sur une échelle numérique. Un score de zéro indique qu'il n'y a pas de calcification sur le trajet des artères coronaires. Par exemple, un score calcique des artères coronaires peut être obtenu par le procédé tel que décrit dans Noninvasive définition of anatomic coronary artery disease by ultrafast computed tomographic scanning: a quantitative pathologie comparison study. Simons DB, Schwartz RS, Edwards WD, Sheedy PF, Breen JF, Rumberger JA. J Am Coll Cardiol. 1992 Nov 1;20(5):1118-26. doi: 10.1016/0735-1097(92)90367-v. PMID: 1401612 ou dans Cardiac calcification as a marker of subclinical atherosclerosis and predictor of cardiovascular events: A review of the evidence. Faggiano P, Dasseni N, Gaibazzi N, Rossi A, Henein M, Pressman G. Eur J Prev Cardiol. 2019 Jul;26(11):1191-1204. doi: 10.1177/2047487319830485. Epub 2019 Mar 7. PMID: 30845832 ou dans Mortensen MB, Cainzos-Achirica M, Steffensen FH, Bratker HE, Jensen JM, Sand NPR, Maeng M, Bruun JM, Blaha MJ, Srarensen HT, Pareek M, Nasir K, Norgaard BL. Association of Coronary Plaque With Low-Density Lipoprotein Cholesterol Levels and Rates of Cardiovascular Disease Events Among Symptomatic Adults.

Les valeurs de CAC score sont corrélées au risque de CAD. En d'autres termes le risque cardiovasculaire peut être évalué en fonction de la valeur du CAC score. En analysant leurs distributions au sein des deux sous-échantillons, on observe que chez les « non-mutés » (Fig. 10B) les valeurs médianes de CAC score les plus basses (moindre risque de CAD) sont retrouvées chez les patients des 5 derniers déciles (6, Jaune à 10, rouge foncé) alors que les patients du 1^{er} décile (1, bleu foncé) présentent les valeurs plus élevées (plus fort risque de CAD).

La Fig. 11 montre la distribution des moyennes de variation des valeurs de LDLc au cours du suivi (% de réduction), cette variation étant évaluée entre 2 moments au cours du suivi du patient. La distribution est déterminée en fonction des déciles de prédiction de distribution des patients « mutés » (Fig. 11A, n = 1061 individus) et « non-mutés » (Fig. 11B, n = 961 individus) selon les valeurs de LDLc et de Score PG, avec les mêmes échelles de LDLc et de Score PG que celles pour les distributions des figures 6 à 10.

Pour ce qui est du pourcentage de variation des valeurs de LDLc au cours du suivi, on observe que les patients « non-mutés » du 1^{er} décile (1, bleu foncé, Fig. 11B) se comportent (-50% de réduction) comme les patients « mutés » des déciles les plus élevés (8, orange foncé à 10, rouge foncé, Fig. 11A).

### Conclusion et perspectives

Sur la base des résultats décrits ci-dessus, une méthode permettant une nouvelle classification des hypercholestérolémies sévères fondée sur le modèle statistique de régression (Fig. 8 et 9) est proposée.

Cette méthode conduit à l'individualisation de classes d'individus ayant des caractéristiques génétiques mais aussi clinico-biologiques distinctes.

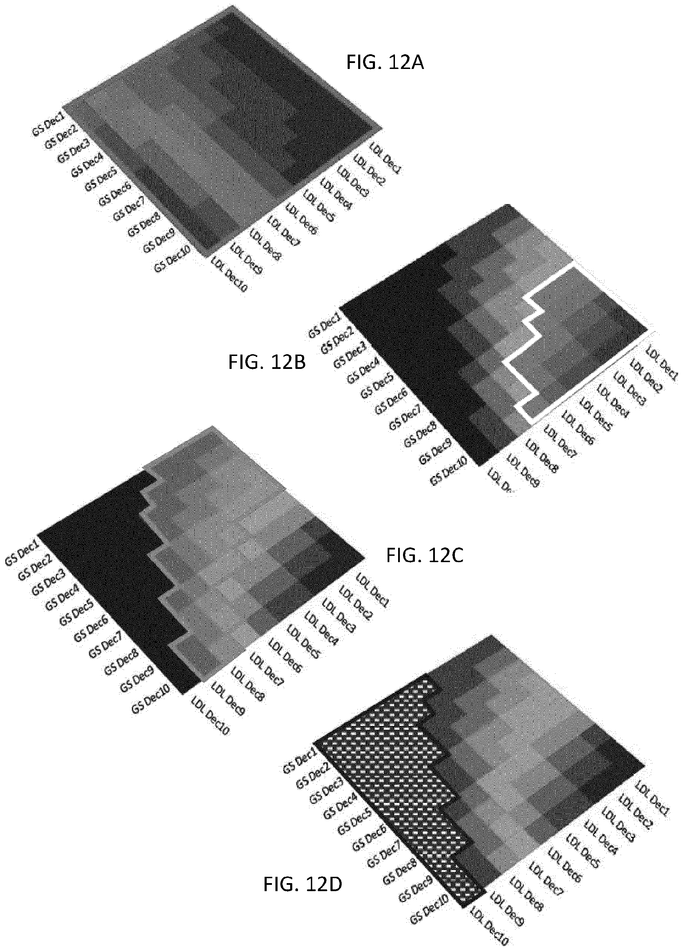

La Fig. 12 illustre la méthode d'obtention des classes sur la base des distributions statistiques des figures 8 et 9 selon un exemple de réalisation utilisant des échelles en déciles. Des échelles en percentiles ou autres quantiles peuvent également être utilisées pour la concentration en LDLc et/ou le score PG et/ou la proportion de la population. Elle repose sur la définition de déciles de distribution cumulée selon les modèles prédictifs précédemment décrits, pour les individus « mutés » et « non mutés », respectivement. Selon ces distributions, environ 10% des individus appartiennent à chaque décile (aux erreurs d'arrondi près dans le calcul des déciles). On peut s'attendre à ce que, dans la population des patients auxquels ces distributions seront appliquées, la répartition soit identique ou similaire.

Une première classe est la classe des individus « mutés ». Cette première classe correspond aux déciles de 1 à 10 précédemment décrits du sous-échantillon d'individus « mutés » (Fig. 9A). Cette première classe correspond au sous-échantillon d'individus « mutés », dont les couples de valeurs de LDLc et de Score PG se trouvent tous dans le cadre rouge en Fig. 12A. Des sous-catégories peuvent être définies dans cette première classe, notamment sur la base de la distribution statistique de la Fig. 10A (individus « mutés »), en fonction des niveaux de risque obtenus pour cette classe.

Une deuxième classe est définie pour les individus « non-mutés ». Cette deuxième classe correspond aux 5 déciles les plus élevés (déciles de 6 à 10, soit environ 50% de la distribution de la Fig. 9B) du sous-échantillon d'individus « non-mutés » (Fig. 9B). Cette deuxième classe correspond aux individus « non-mutés » dont les couples de valeurs de LDLc et de Score PG se trouvent dans le cadre blanc en Fig. 12B.

Cette deuxième classe est une classe dite des individus à hérédité polygénique en ce que :
leur hypercholestérolémie, bien que sévère, correspond à la première moitié des déciles de LDLc (les plus bas) et que leur Score PG est le plus élevé (deuxième moitié des déciles : 6 à 10) signifiant leur association à une forte combinatoire des variants génétiques du Score PG (cf. : [01);
leur distribution selon le modèle statistique prédictif montre un biais de répartition dans la mesure où cette classe d'individus qui représente 50% des patients « non-mutés » ne se répartie que sur 26% de la distribution.
leur risque cardiovasculaire, tel que apprécié par leurs valeurs de CAC, montre des valeurs médianes de CAC score les plus basses pour les patients de cette classe ce qui correspond au risque cardiovasculaire le plus bas.

Une troisième classe est définie pour les individus « non-mutés ». Cette troisième classe correspond aux 4 déciles moyens (déciles de 2 à 5) du sous-échantillon d'« non-mutés » (Fig. 9B). Cette troisième classe correspond aux individus « non-mutés » dont les couples de valeurs de LDLc et de Score PG se trouvent dans le cadre vert en Fig. 12C.

Cette troisième classe est la classe dite des individus à hérédité indéterminée en ce que :
leur hypercholestérolémie se répartie sur la presque totalité des déciles de LDLc (1 à 9) et sur l'ensemble des déciles de Score PG (1 à 10) ne montrant aucune association particulière avec la combinatoire des variants génétiques du score PG (cf. : [01) ;
leur distribution selon le modèle statistique prédictif ne montre aucun biais de répartition dans la mesure où cette classe d'individus qui représente 40% des patients « non-mutés » se répartie également sur 45% de la distribution.

Une quatrième classe est définie pour les individus « non-mutés ». Cette quatrième classe correspond au 1^{er} décile (décile 1) du sous-échantillon d'individus « non-mutés » (Fig. 9B). Cette quatrième classe correspond aux individus « non-mutés » dont les couples de valeurs de LDLc et de Score PG se trouvent dans le cadre bleu en Fig. 12D.

Cette quatrième classe est la classe dite des individus à hérédité monogénique dont la cause reste à identifier en ce que :
leur hypercholestérolémie sévère se répartie sur les déciles les plus élevés de LDLc (5 à 10) et sur l'ensemble des déciles de Score PG (1 à 10) ne montrant aucune association particulière avec la combinatoire des variants génétiques du score PG ;
leur distribution selon le modèle statistique prédictif montre un biais de répartition dans la mesure où cette classe d'individus qui ne représente que 10% des patients « non-mutés » occupe 29% de la distribution ;
leur risque cardiovasculaire, tel que apprécié par leurs valeurs de CAC, ainsi que les variations de leurs valeurs de LDLc au cours du suivi (% de réduction) montrent des valeurs les plus élevées pour les patients de cette classe correspondant à celles retrouvées chez les patients « mutés ».

Il est possible de prédire, sur la base du couple de valeur de LDLc et du score PG obtenu pour un patient et/ou de la classe d'appartenance d'un individu et de la distribution de la Fig. 11B, un taux de variation de la concentration de cholestérol dans les lipoprotéines de basse densité. Cette variation peut être la conséquence d'un traitement médicamenteux du patient ou être liée à un autre facteur. Ce taux de variation correspond par exemple à une valeur moyenne, minimale ou maximale du taux de réduction calculée sur la base des valeurs des taux de réduction des points de la distribution de la Fig. 11B qui se trouvent dans le cadre (voir Fig. 12B à 12D) correspondant à la classe concernée.

Il est possible, sur la base du couple de valeur de LDLc et du score PG obtenu pour un patient et/ou de la classe d'appartenance d'un individu et par l'intermédiaire de la distribution des médianes de CAC de la Fig. 10B, de prédire un niveau de risque d'accident cardiovasculaire des patients. Ce niveau de risque correspond par exemple à une valeur CAC moyenne, minimale ou maximale, calculée sur la base des valeurs de CAC des points de la distribution de la Fig. 10B qui se trouvent dans le cadre (voir Fig. 12B à 12D) correspondant à la classe concernée.

Il est possible, pour chacune des classes d'individus par l'intermédiaire de la distribution des médianes de CAC de la Fig. 10A, de prédire un niveau de risque d'accident cardiovasculaire des patients appartenant à cette classe. Ce niveau de risque correspond par exemple à une valeur CAC moyenne, minimale ou maximale ou une plage de valeurs, calculée sur la base des valeurs de CAC des points de la distribution de la Fig. 10A qui se trouvent dans le cadre (voir Fig. 12A) correspondant à la classe concernée.

Le niveau de risque prédit pour un patient peut aussi être calculé comme la valeur médiane du score CAC fourni par la distribution statistique des scores CAC (fig. 10) obtenus pour le sous-ensemble d'individus ayant le même niveau (exprimé en déciles) de score de risque polygénique que le patient et le même niveau de concentration de cholestérol dans des lipoprotéines de basse densité, ce sous-ensemble d'individus étant soit un sous-ensemble du premier sous-échantillon d'individus « mutés » si le patient est lui-même un individu « muté », soit un sous-ensemble du deuxième sous-échantillon d'individus « non-mutés » si le patient est lui-même un individu «non- muté ».

En effet, pour les patients actuellement considérés comme « non mutés », qui représentent plus de 70% de nos diagnostics moléculaires en 2022 (Fig. 3), il devient possible, pour la moitié d'entre eux (cf. : déciles 6 à 10, Fig. 9B) de les informer de leur hérédité polygénique dont le risque de CAD associé semble intermédiaire entre celui des formes monogéniques et non héréditaires.

Un élargissement des analyses moléculaires peut être proposé aux patients de la quatrième (1^{er} décile de la Fig. 9B) dont les caractéristiques suggèrent qu'ils se comportent comme des patients à hérédité monogénique mais dont l'étiologie moléculaire reste à identifier.

Enfin la clarification de la distinction entre les différentes étiologies d'hypercholestérolémies sévères permet de mieux caractériser les variations de leurs valeurs de LDLc au cours du suivi et leurs risques cardiovasculaires respectifs pour une meilleure prise en charge médicale des patients.

Dans la présente par « accident cardiovasculaire » on entend tout accident cardiovasculaire connu de la personne du métier. Il peut s'agir par exemple d'une coronaropathie, d'un accident vasculaire cérébral ou de toute complication vasculaire liée à l'athérosclérose connue de la personne du métier. Il peut s'agir par exemple d'un accident cardiovasculaire tel que décrit dans « The risk of various types of cardiovascular diseases in mutation positive familial hypercholesterolemia; a review. » Hovland A, Mundal LJ, Veierød MB, Holven KB, Bogsrud MP, Tell GS, Leren TP, Retterstral K. Front Genet. 2022 Dec 6;13:1072108. doi: 10.3389/fgene.2022.1072108. PMID: 36561318; PMCID: PMC9763610.

Dans la présente par « risque cardiovasculaire », on entend tout risque cardiovasculaire connu de la personne du métier. Il peut s'agir par exemple d'un risque cardiovasculaire tel que décrit dans « The risk of various types of cardiovascular diseases in mutation positive familial hypercholesterolemia; a review. » Hovland A, Mundal LJ, Veierød MB, Holven KB, Bogsrud MP, Tell GS, Leren TP, Retterstral K. Front Genet. 2022 Dec 6;13:1072108. doi: 10.3389/fgene.2022.1072108. PMID: 36561318; PMCID: PMC9763610. Dans la présente le risque cardiovasculaire peut, par exemple, être déterminé par la valeur du score CAC.

Dans la présente par « traitement de l'hypercholestérolémie» on entend tout médicament adapté pour le traitement de l'hypercholestérolémie connu de la personne du métier et/ou disponible dans le commerce. Il peut s'agir par exemple d'un médicament pour le traitement de l'hypercholestérolémie, par exemple de tout médicament contre l'excès de cholestérol tel que décrit dans le dictionnaire Vidal et/ou disponible dans le commerce. Il peut s'agir par exemple d'un médicament choisi dans le groupe comprenant les médicaments de la classe des statines, par exemple atorvastatine, rosuvastatine, pitavastatine, pravastatine, simvastatine, des fibrates, par exemple le bézafibrate, ciprofibrate, fénofibrate, des inhibiteurs de l'absorption du cholestérol ou des acides biliaires, par exemple l'ézétimibe, colestyramine, des inhibiteurs de l'action de PCSK9, par exemple l'Alirocumab, Evolocumab. Il peut s'agir par exemple de régimes diététiques adaptés, par exemple d'un régime alimentaire et /ou diététique tel que décrit dans « Recent advances in the management and implementation of care for familial hypercholesterolaemia ». Lan NSR, Bajaj A, Watts GF, Cuchel M. Pharmacol Res. 2023 Aug;194:106857. doi: 10.1016/j.phrs.2023.106857. Epub 2023 Jul 17. PMID: 37460004, et/ou « Advances in Treatment of Dyslipidemia ». Dybiec J, Baran W, D 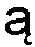 bek B, Fularski P, M 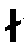 ynarska E, Radzioch E, Rysz J, Franczyk B. Int J Mol Sci. 2023 Aug 27;24(17):13288. doi: 1.0.3390/ijms241713288. PMID: 37686091; PMCID: PMC10488025., de LDL aphérèses, par exemple tel que décrites dans Lipoprotein Apheresis. Feingold KR. 2023 Feb 19. In: Feingold KR, Anawalt B, Blackman MR, Boyce A, Chrousos G, Corpas E, de Herder WW, Dhatariya K, Dungan K, Hofland J, Kalra S, Kaltsas G, Kapoor N, Koch C, Kopp P, Korbonits M, Kovacs CS, Kuohung W, Laferrère B, Levy M, McGee EA, McLachlan R, New M, Purnell J, Sahay R, Shah AS, Singer F, Sperling MA, Stratakis CA, Trence DL, Wilson DP, editors. Endotext [Internet]. South Dartmouth (MA): MDText.com, Inc.; 2000-. PMID: 28402616, de procédés pharmacologiques en cours de développement, par exemple tel que décrits dans Current Options and Future Perspectives in the Treatment of Dyslipidemia. Muscoli S, Ifrim M, Russo M, Candido F, Sanseviero A, Milite M, Di Luozzo M, Marchei M, Sangiorgi GM. Current Options and Future Perspectives in the Treatment of Dyslipidemia. J Clin Med. 2022 Aug 12;11(16):4716. doi: 10.3390/jcm11164716. PMID: 36012957; PMCID: PMC9410330.

Les informations fournies par les distributions de la figure 8 ou 9 permettent notamment de prédire une classe d'appartenance du patient comme décrit par rapport à la figure 12.

Les informations fournies par les distributions des figures 10 et 11 permettent de prédire, ou au moins estimer, pour un patient, sur la base du couple de valeurs LDLC et score PG obtenu pour ce patient, un niveau de risque d'accident cardiovasculaire et/ou une variation des valeurs de LDLc.

Ainsi, un profil hypercholestérolémique peut être déterminé pour le patient. Un profil hypercholestérolémique correspond ici à une catégorie ou un statut de patient hypercholestérolémique sévère. Cette catégorie peut être déterminée sur la base de d'un ou plusieurs des paramètres considérés ici, à savoir:
- la classe du patient (selon ce qui est décrit par exemple par référence à la FIG. 12); et/ou
- le niveau de risque d'accident cardiovasculaire estimé ; et/ou
- une variation des valeurs de LDLc en cours de suivi.

Chacun de ces paramètres est lié au couple de valeurs de LDLc et score PG obtenu pour le patient: ce couple de valeurs détermine une position dans la distribution statistique du sous-échantillon d'individus « mutés » si le patient est un individu « muté ») (ou respectivement des individus «non-mutés » si le patient est un individu « non-muté »), et donc un sous-ensemble d'individus dans le sous-échantillon d'individus « mutés » (ou respectivement des individus «non-mutés ») présentant le même couple (exprimé en déciles) de valeur de LDLc et score PG que celui obtenu pour le patient.

En considérant la classe du patient :
- la première classe correspond à un profil hypercholestérolémique avec hérédité dite monogénique confirmée,
- la deuxième classe correspond à un profil hypercholestérolémique avec hérédité dite polygénique,
- la troisième classe correspond à un profil hypercholestérolémique avec hérédité dite indéterminée,
- la quatrième classe correspond à un profil hypercholestérolémique avec hérédité dite monogénique de cause non identifiée ;

Au sein de la première classe, on peut déterminer des catégories de patients selon le couple de valeurs de LDLc et score PG obtenu pour le patient, et/ou selon autre paramètre tel que le niveau de risque d'accident cardiovasculaire estimé et/ou une variation des valeurs de LDLc.

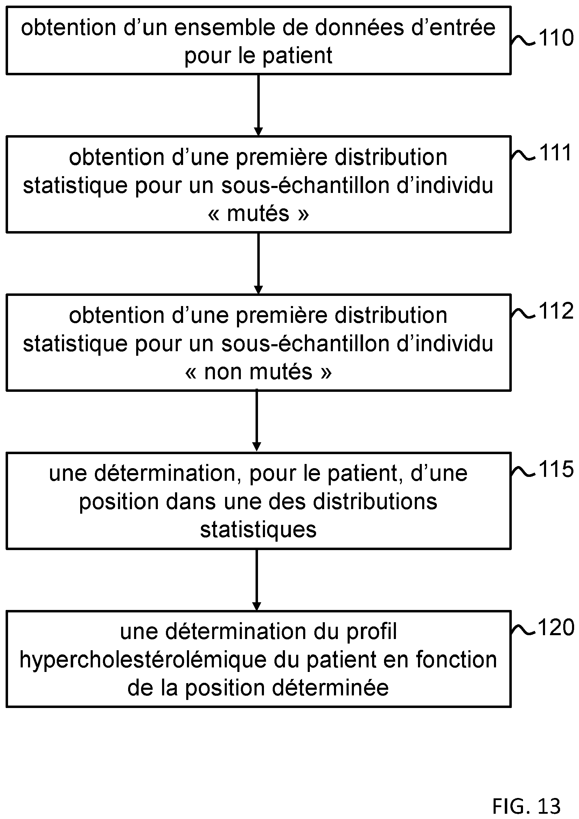

La Fig. 13 montre un organigramme d'un procédé de classification selon un ou plusieurs exemples de réalisation.

Les étapes du procédé peuvent être mises en oeuvre par dispositif informatique selon un des exemples décrits ici.

Bien que les étapes soient décrites de manière séquentielle, la personne du métier appréciera que certaines étapes puissent être omises, combinées, exécutées dans un ordre différent et/ou en parallèle.

Le procédé est un procédé in vitro de détermination d'un profil hypercholestérolémique pour un patient hypercholestérolémique sévère. Le procédé peut être mis en oeuvre par ordinateur.

Dans une étape 110, le procédé comprend une obtention d'un ensemble de données d'entrée pour le patient. Cette obtention peut se faire par lecture dans une mémoire de ces données d'entrée.

Les données d'entrée comprennent, pour le patient :
- une information génétique sur la présence ou non d'une mutation génétique induisant une hypercholestérolémie pour au moins un gène parmi un ensemble de gènes.
- un score de risque polygénique associé à une hypercholestérolémie.
- une concentration de cholestérol dans les lipoprotéines de basse densité, en absence de traitement.

Dans une étape 111, le procédé comprend une obtention d'une première distribution statistique en fonction d'une échelle de niveaux de score de risque polygénique et d'une échelle de niveaux de concentration de cholestérol dans des lipoprotéines de basse densité. La première distribution statistique donnant en fonction de ces échelles une répartition des individus d'un premier sous-échantillon d'individus hypercholestérolémiques sévères présentant au moins une mutation génétique à l'origine d'une hypercholestérolémie pour au moins un gène parmi un ensemble de gènes (sous-échantillon d'individus « mutés »). Cette obtention peut se faire par lecture dans une mémoire de données définissant la première distribution statistique.

L'ensemble des gènes peut comprendre au moins : *LDLR, APOB, PCSK9, APOE.*

Dans une étape 112, le procédé comprend une obtention d'une deuxième distribution statistique en fonction de la même échelle de niveaux du score de risque polygénique et de la même échelle de niveaux de concentration de cholestérol dans les lipoprotéines de basse densité. La deuxième distribution statistique donnant en fonction de ces échelles une répartition des individus d'un deuxième sous-échantillon d'individus hypercholestérolémiques sévères ne présentant pas de mutation génétique dans l'ensemble de gènes. (sous-échantillon d'individus « non-mutés »). Cette obtention peut se faire par lecture dans une mémoire de données définissant la deuxième distribution statistique.

L'échelle de niveaux de concentration de cholestérol dans les lipoprotéines de basse densité utilisée dans les premières et deuxièmes distributions statistiques est obtenue sur la base d'une distribution statistique des concentrations de cholestérol dans les lipoprotéines de basse densité obtenues pour les individus de la population totale constituée des premier et deuxième sous-échantillons d'individus.

L'échelle de niveaux de score de risque polygénique utilisée dans les premières et deuxièmes distributions statistique est obtenue sur la base de la distribution statistique des scores de risque polygénique obtenus pour les individus du premier sous-échantillon d'individus.

La première distribution statistique peut être obtenue par régression à partir d'une distribution statistique initiale obtenue le premier sous-échantillon d'individus.

La deuxième distribution statistique peut être obtenue par régression à partir d'une distribution statistique initiale obtenue le deuxième sous-échantillon d'individus.

L'échelle de niveaux de concentration de cholestérol dans les lipoprotéines de basse densité peut être une échelle en déciles dont les déciles sont calculés sur la base d'une distribution statistique des concentrations de cholestérol dans les lipoprotéines de basse densité obtenues à partir d'échantillon biologiques pour les individus de la population totale constituée des premier et deuxième sous-échantillons d'individus.

L'échelle de niveaux de score de risque polygénique peut être une échelle en déciles dont les déciles sont calculés sur la base de la distribution statistique des scores de risque polygénique obtenus à partir d'échantillons biologiques pour les individus du premier sous-échantillon d'individus.

Les déciles de score polygénique sont définis sur une population générale pour laquelle les sujets au-dessus de 1.9 g.L⁻¹ de LDLc ne représentent que 10% de la population, or il s'agit de l'entièreté de la population d'intérêt. Les individus « mutés » de la population d'intérêt constituent un sous-échantillon représentatif de la distribution aléatoire du score PG dans la population hypercholestérolémique sévère car c'est du fait de la présence d'une mutation qu'ils appartiennent à cette population.

Dans une étape 115, le procédé comprend une détermination, pour le patient, d'une position dans une des distributions statistiques en fonction du score de risque polygénique et de la concentration de cholestérol dans les lipoprotéines de basse densité. La position est déterminée dans la première distribution statistique lorsque l'information génétique obtenue pour le patient indique la présence d'au moins une mutation (patient « muté ») et dans la deuxième distribution lorsque l'information génétique obtenue pour le patient indique l'absence de mutation (patient « non-muté »).

Dans une étape 120, le procédé comprend une détermination du profil hypercholestérolémique du patient en fonction de la position déterminée.

La détermination du profil hypercholestérolémique du patient peut comprendre une détermination d'une classe d'appartenance du patient sur la base de la position obtenue.

La classe peut être
- soit une première classe lorsque l'information génétique indique la présence d'une mutation génétique;
- soit une deuxième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution statistique présentant une valeur supérieure ou égale à un premier seuil ;
- soit une troisième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution statistique indiquent une valeur supérieure ou égale à un deuxième seuil et inférieure strictement au premier seuil;
- soit une quatrième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution indiquent une valeur strictement inférieure au deuxième seuil.

Lorsque les premières et deuxièmes distributions statistiques donnent la répartition des individus en déciles : le premier seuil correspond au 6^{ème} décile et le deuxième seuil correspond au 2^{ème} décile.

L'appartenance à une des classes permet de définir des profils hypercholestérolémiques. La première classe correspond ainsi à un profil hypercholestérolémique avec hérédité dite monogénique confirmée. La deuxième classe correspond à un profil hypercholestérolémique avec hérédité dite polygénique. La troisième classe correspond à un profil hypercholestérolémique avec hérédité dite indéterminée. La quatrième classe correspond à un profil hypercholestérolémique avec hérédité dite monogénique de cause non identifiée.

La détermination du profil hypercholestérolémique du patient peut comprendre une prédiction d'un niveau de risque d'accident cardiovasculaire pour le patient sur la base de la position obtenue et d'une troisième distribution statistique (cf. : fig. 10) fonction de la même échelle de niveaux de score de risque polygénique et de la même échelle de niveaux de concentration de cholestérol dans des lipoprotéines de basse densité, la troisième distribution statistique donnant en fonction de ces échelles un niveau de risque d'accident cardiovasculaire.

Le niveau de risque est calculé comme une valeur médiane du score CAC, Coronary Artery Calcium de la distribution statistique des scores CAC obtenus pour le sous-ensemble d'individus ayant le même niveau de score de risque polygénique que le patient et le même niveau de concentration de cholestérol dans des lipoprotéines de basse densité, ce sous-ensemble d'individus étant soit un sous-ensemble du premier sous-échantillon d'individus « mutés » lorsque l'information génétique obtenue pour le patient indique la présence d'au moins une mutation, soit un sous-ensemble du deuxième sous-échantillon d'individus « non-mutés » lorsque l'information génétique obtenue pour le patient indique l'absence de mutation.

Une ou plusieurs ou toutes les étapes d'un ou des procédés décrits dans ce document peuvent être mises en oeuvre par un logiciel ou programme d'ordinateur et/ou par du hardware, par exemple par circuit, programmable ou non, spécifique ou non.

Les fonctions, étapes et procédés décrits dans ce document peuvent être mises en oeuvre par logiciel (par exemple, via un logiciel sur un ou plusieurs processeurs, pour exécution sur un ordinateur à usage général ou à usage spécifique) et/ou être mises en oeuvre par du hardware (par exemple un ou plusieurs circuits, et/ou tout autre composant matériel, qu'il soit à base d'électronique, d'optique, ou d'autres technologies).

La présente description concerne ainsi un logiciel ou programme d'ordinateur, susceptible d'être exécuté par un dispositif hôte, au moyen d'un ou plusieurs processeurs de données, ce logiciel / programme comportant des instructions pour causer l'exécution par ce dispositif hôte de tout ou partie des étapes de l'un ou des procédés décrits dans ce document. Ces instructions sont destinées à être stockées dans une mémoire du dispositif hôte, chargées puis exécutées par un ou plusieurs processeurs de ce dispositif hôte de sorte à causer l'exécution par ce dispositif hôte du procédé considéré.

Ce logiciel / programme peut être codé au moyen de n'importe quel langage de programmation, et être sous la forme de code source, code objet, ou de code intermédiaire entre code source et code objet, tel que dans une forme partiellement compilée, ou dans n'importe quelle autre forme souhaitable.

Le dispositif hôte peut être mis en oeuvre par une ou plusieurs machines physiquement distinctes. Le dispositif hôte peut présenter globalement l'architecture d'un ordinateur, incluant un ou des constituants d'une telle architecture : mémoire(s) de données, processeur(s), bus de communication, interface(s) matérielle(s) pour la connexion de ce dispositif hôte à un réseau ou un autre équipement, interface(s) utilisateur, etc.

Dans un mode de réalisation, tout ou partie des étapes d'un procédé de déclenchement d'opération ou d'un autre procédé décrit dans ce document sont mises en oeuvre par un dispositif doté de moyens de mise en oeuvre de ces étapes de ce procédé.

Ces moyens peuvent comprendre des moyens logiciels (software) (par exemple, des instructions d'un ou plusieurs composants d'un programme) et/ou moyens matériels (hardware) (par exemple, circuit(s), mémoire(s) de données, processeur(s), bus de communication, interface(s) matérielle(s), etc).

Ces moyens peuvent comprendre par exemple un ou plusieurs circuits configurés pour exécuter une ou plusieurs ou toutes les étapes d'un des procédés décrits ici. Ces moyens peuvent comprendre par exemple au moins un processeur et au moins une mémoire comprenant des instructions de programme configurées pour, lorsqu'elles sont exécutées par le processeur, causer l'exécution par le dispositif d'une ou plusieurs ou toutes les étapes d'un des procédés décrits ici.

Des moyens mettant en oeuvre une fonction ou un ensemble de fonctions peuvent correspondre dans ce document à un composant logiciel, à un composant matériel ou bien à une combinaison de composants matériels et/ou logiciels, apte à mettre en oeuvre la fonction ou l'ensemble de fonctions, selon ce qui est décrit ci-dessous pour les moyens concernés.

La présente description concerne aussi un support d'informations lisible par un processeur de données, et comportant des instructions d'un programme tel que mentionné ci-dessus.

Le support d'informations peut être n'importe quel moyen matériel, entité ou dispositif, capable de stocker les instructions d'un programme tel que mentionné ci-dessus. Les supports de stockage de programme utilisables incluent les mémoires ROM ou RAM, les supports de stockage magnétiques tels que des disques magnétiques et des bandes magnétiques, les disques durs ou des supports de stockage de données numériques à lecture optique, ou toute combinaison de ces supports.

Dans certains cas, le support de stockage lisible par ordinateur n'est pas transitoire. Dans d'autres cas, le support d'informations peut être un support transitoire (par exemple, une onde porteuse) pour la transmission d'un signal (signal électromagnétique, électrique, radio ou optique) porteur des instructions de programme. Ce signal peut être acheminé via un moyen de transmission approprié, filaire ou non filaire : câble électrique ou optique, liaison radio ou infrarouge, ou par d'autres moyens.

Un mode de réalisation concerne également un produit programme d'ordinateur comprenant un support de stockage lisible par ordinateur sur lequel sont stockées des instructions de programme, les instructions de programme étant configurées pour causer la mise en oeuvre par le dispositif hôte (par exemple un ordinateur) de tout ou partie des étapes d'un ou des procédés décrits ici lorsque les instructions de programme sont exécutées par un ou plusieurs processeurs et/ou un ou plusieurs composants matériels programmables du dispositif hôte.

Dans la présente description, les termes "moyens configurés pour exécuter une ou plusieurs fonctions" ou "moyens pour exécuter une ou plusieurs fonctions" peuvent correspondre à un ou plusieurs blocs fonctionnels comprenant des circuits adaptés pour exécuter ou configurés pour exécuter la ou les fonctions concernées. Le bloc peut exécuter lui-même cette fonction ou coopérer et/ou communiquer avec d'autres blocs pour exécuter cette fonction. Les "moyens" peuvent correspondre à ou être mis en oeuvre comme "un ou plusieurs modules", "un ou plusieurs dispositifs", "une ou plusieurs unités", etc.

Les moyens peuvent comprendre au moins un processeur et au moins une mémoire comprenant au moins une mémoire stockant des instructions qui, lorsqu'elles sont exécutées par au moins un processeur, amènent l'appareil à la ou aux fonctions considérées. Les moyens peuvent comprendre des circuits (par exemple, des circuits de traitement) configurés pour exécuter la ou les fonctions considérées.

Bien que les termes premier, second, etc. puissent être utilisés ici pour décrire divers éléments, ces éléments ne doivent pas être limités par ces termes. Ces termes ne sont utilisés que pour distinguer un élément d'un autre. Par exemple, un premier élément peut être appelé second élément, et de la même manière, un second élément peut être appelé premier élément, sans sortir du champ d'application de la présente divulgation. Dans le présent document, le terme "et/ou" comprend toutes les combinaisons d'un ou de plusieurs des éléments énumérés.

La terminologie utilisée ici a pour seul but de décrire des modes de réalisation particuliers et n'est pas limitative. Dans le présent document, les formes singulières "a", "un" et "le" s'entendent également au pluriel, à moins que le contexte n'indique clairement le contraire. Il est entendu que les termes "comprend", "comprend", "inclut" et/ou "y compris", lorsqu'ils sont utilisés ici, précisent la présence de caractéristiques, de nombres entiers, d'étapes, d'opérations, d'éléments et/ou de composants donnés, mais n'excluent pas la présence ou l'ajout d'une ou de plusieurs autres caractéristiques, nombres entiers, étapes, opérations, éléments, composants et/ou groupes de ces caractéristiques, nombres entiers, étapes, opérations, éléments, composants et/ou groupes de ces éléments.

Bien que certains aspects de la présente divulgation aient été particulièrement illustrés et décrits en référence aux réalisations ci-dessus, la personne du métier comprendra que divers modes de réalisations supplémentaires peuvent être envisagées en modifiant les dispositifs et procédés décrits.

### LISTE DES PRINCIPALES ABRÉVIATIONS

- CAC: Coronary Artery Disease
- DLCN: Dutch Lipid Clinic Network
- HF: hypercholestérolémie familiale
- NGS: Next Génération Sequencing
- SNP: Single Nucléotide Polymorphism

### LISTE DES REFERENCES CITEES

[01] Use of low-density lipoprotein cholestérol gène score to distinguish patients with polygenic and monogenic familial hypercholesterolaemia: a case-control study". Talmud PJ, Shah S, Whittall R, Futema M, Howard P, Cooper JA, Harrison SC, Li K, Drenos F, Karpe F, Neil HA, Descamps OS, Langenberg C, Lench N, Kivimaki M, Whittaker J, Hingorani AD, Kumari M, Humphries SE.Lancet. 2013 Apr 13;381(9874):1293-301. PMID: 23433573

## Revendications

1. Procédé *in-vitro* de détermination d'un profil hypercholestérolémique pour un patient hypercholestérolémique sévère, le procédé étant mis en oeuvre par ordinateur et comprenant :
- une obtention d'une première distribution statistique en fonction d'une échelle de niveaux de score de risque polygénique et d'une échelle de niveaux de concentration de cholestérol dans des lipoprotéines de basse densité, la première distribution statistique donnant en fonction de ces échelles une répartition des individus d'un premier sous-échantillon d'individus hypercholestérolémiques sévères présentant au moins une mutation génétique à l'origine d'une hypercholestérolémie pour au moins un gène parmi un ensemble de gènes;
- une obtention d'une deuxième distribution statistique en fonction de la même échelle de niveaux du score de risque polygénique et de la même échelle de niveaux de concentration de cholestérol dans les lipoprotéines de basse densité, la deuxième distribution statistique donnant en fonction de ces échelles une répartition des individus d'un deuxième sous-échantillon d'individus hypercholestérolémiques sévères ne présentant pas de mutation génétique dans l'ensemble de gènes;
- une obtention, par lecture dans une mémoire, de données déterminées à partir d'au moins un échantillon biologique pour le patient, les données comprenant :
∘ une information génétique sur la présence ou non d'au moins une mutation génétique à l'origine d'une hypercholestérolémie pour au moins un gène parmi l'ensemble de gènes;
∘ un score de risque polygénique ;
∘ une concentration de cholestérol dans les lipoprotéines de basse densité en absence de traitement;
- une détermination, pour le patient, d'une position dans une des distributions statistiques en fonction du score de risque polygénique et de la concentration de cholestérol dans les lipoprotéines de basse densité, la position étant déterminée dans la première distribution statistique lorsque l'information génétique obtenue pour le patient indique la présence d'au moins une mutation et dans la deuxième distribution lorsque l'information génétique obtenue pour le patient indique l'absence de mutation ;
- une détermination du profil hypercholestérolémique du patient en fonction de la position déterminée ;
l'échelle de niveaux de concentration de cholestérol dans les lipoprotéines de basse densité étant obtenue sur la base d'une distribution statistique des concentrations de cholestérol dans les lipoprotéines de basse densité obtenues pour les individus de la population totale constituée des premier et deuxième sous-échantillons d'individus;
l'échelle de niveaux de score de risque polygénique étant obtenue sur la base de la distribution statistique des scores de risque polygénique obtenus pour les individus du premier sous-échantillon d'individus.

2. Procédé selon la revendication 1, dans lequel la détermination du profil hypercholestérolémique du patient comprend une détermination d'une classe d'appartenance du patient sur la base de la position obtenue, la classe étant
- soit une première classe lorsque l'information génétique indique la présence d'une mutation génétique;
- soit une deuxième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution statistique présentant une valeur supérieure ou égale à un premier seuil ;
- soit une troisième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution statistique indiquent une valeur supérieure ou égale à un deuxième seuil et inférieure strictement au premier seuil;
- soit une quatrième classe lorsque l'information génétique indique l'absence d'une mutation génétique et que les valeurs du score polygénique et de la concentration de cholestérol dans les lipoprotéines de basse densité obtenues pour le patient correspondent à une position dans la deuxième distribution indiquent une valeur strictement inférieure au deuxième seuil.

3. Procédé selon l'une quelconque des revendications précédentes, dans lequel la première classe correspond à un profil hypercholestérolémique avec hérédité dite monogénique confirmée, dans lequel la deuxième classe correspond à un profil hypercholestérolémique avec hérédité dite polygénique, dans lequel la troisième classe correspond à un profil hypercholestérolémique avec hérédité dite indéterminée, dans lequel la quatrième classe correspond à un profil hypercholestérolémique avec hérédité dite monogénique de cause non identifiée.

4. Procédé selon l'une quelconque des revendications précédentes, dans lequel la première distribution statistique est obtenue par régression de type modèle additif généralisé à partir d'une distribution statistique initiale obtenue le premier sous-échantillon d'individus.

5. Procédé selon l'une quelconque des revendications précédentes, dans lequel la deuxième distribution statistique est obtenue par régression de type modèle additif généralisé à partir d'une distribution statistique initiale obtenue le deuxième sous-échantillon d'individus.

6. Procédé selon l'une quelconque des revendications précédentes, dans lequel l'échelle de niveaux de concentration de cholestérol dans les lipoprotéines de basse densité est une échelle en déciles dont les déciles sont calculés sur la base d'une distribution statistique des concentrations de cholestérol dans les lipoprotéines de basse densité obtenues à partir d'échantillon biologiques pour les individus de la population totale constituée des premier et deuxième sous-échantillons d'individus.

7. Procédé selon l'une quelconque des revendications précédentes, dans lequel l'échelle de niveaux de score de risque polygénique est une échelle en déciles dont les déciles sont calculés sur la base de la distribution statistique des scores de risque polygénique obtenus à partir d'échantillons biologiques pour les individus du premier sous-échantillon d'individus.

8. Procédé selon la revendication selon l'une quelconque des revendications 2 à 7, dans lequel les première et deuxième distributions statistiques donnent la répartition des individus en déciles.

9. Procédé selon la revendication 8, dans lequel le deuxième seuil correspond au 2^{ème} décile.

10. Procédé selon la revendication 8 ou 9, dans lequel le deuxième seuil correspond au 2^{ème} décile.

11. Procédé selon l'une quelconque des revendications précédentes, dans lequel l'ensemble des gènes comprend au moins : *LDLR, APOB, PCSK9, APOE.*

12. Procédé selon l'une quelconque des revendications 1 à 11, dans lequel la détermination du profil hypercholestérolémique du patient comprend :
- une prédiction d'un niveau de risque d'accident cardiovasculaire pour le patient sur la base de la position obtenue et d'une troisième distribution statistique fonction de la même échelle de niveaux de score de risque polygénique et de la même échelle de niveaux de concentration de cholestérol dans des lipoprotéines de basse densité, la troisième distribution statistique donnant en fonction de ces échelles un niveau de risque d'accident cardiovasculaire.

13. Procédé selon la revendication 12 dans lequel le niveau de risque est calculé dans la troisième distribution statistique comme une valeur médiane du score CAC, Coronary Artery Calcium, de la distribution statistique des scores CAC obtenus pour le sous-ensemble d'individus ayant le même niveau de score de risque polygénique que le patient et le même niveau de concentration de cholestérol dans des lipoprotéines de basse densité, ce sous-ensemble d'individus étant soit un sous-ensemble du premier sous-échantillon d'individus lorsque l'information génétique obtenue pour le patient indique la présence d'au moins une mutation, soit un sous-ensemble du deuxième sous-échantillon d'individus lorsque l'information génétique obtenue pour le patient indique l'absence de mutation.

14. Un dispositif comprenant des moyens pour mettre en oeuvre les étapes d'un procédé selon l'une quelconque des revendications précédentes.

15. Dispositif selon la revendication 14, dans lequel les moyens comprennent
- au moins un processeur ;
- au moins une mémoire stockant des instructions qui, lorsqu'elles sont exécutées par au moins un processeur, cause l'exécution du procédé par le dispositif.
